(19) **Europäisches Patentamt / European Patent Office / Office européen des brevets**

(11) **EP 2 905 621 B1**

(12) # EUROPEAN PATENT SPECIFICATION

(45) Date of publication and mention
of the grant of the patent:
**02.05.2018 Bulletin 2018/18**

(51) Int Cl.:
***G01N 33/66*** *(2006.01)*

(21) Application number: **15153928.5**

(22) Date of filing: **05.02.2015**

(54) **MEANS AND METHODS FOR DIAGNOSING AND TREATING CDG CAUSED BY A DEFICIENCY OF PGM1**

Mittel und Verfahren zur Diagnose und Behandlung von durch einen PGM1-Mangel verursachtes CDG

Moyens et procédés pour diagnostiquer et traiter l'anomalie congénitale de la glycosylation (CDG) causée par une déficience de PGM1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.02.2014 EP 14000424**

(43) Date of publication of application:
**12.08.2015 Bulletin 2015/33**

(73) Proprietor: **Westfälische Wilhelms-Universität Münster**
**48149 Münster (DE)**

(72) Inventors:
 • **MARQUARDT, Thorsten**
  **48149 Muenster (DE)**
 • **RUST, Stephan**
  **48149 Muenster (DE)**
 • **TEGTMEYER, Laura**
  **48149 Muenster (DE)**
 • **FINGERHUT, Ralph**
  **8032 Zuerich (CH)**

(74) Representative: **Schiweck, Weinzierl & Koch Patentanwälte Partnerschaft mbB**
**Landsberger Straße 98**
**80339 München (DE)**

(56) References cited:
 • BIFFI ET AL: "Carbohydrate-deficient transferrin (CDT) as a biochemical tool for the screening of congenital disorders of glycosylation (CDGs)", CLINICAL BIOCHEMISTRY, ELSEVIER INC, US, CA, vol. 40, no. 18, 26 November 2007 (2007-11-26), pages 1431-1434, XP022361942, ISSN: 0009-9120, DOI: 10.1016/J.CLINBIOCHEM.2007.08.015
 • FINGERHUT R ET AL: "DETERMINATION OF PHOSPHOGLUCOMUTASE (PGM)- AND GLUCOSE-6-PHOSPHATE DEHYDROGENASE (G6PDH) ACTIVITY FROM DRIED BLOOD SPOTS (DBS) BY A SIMPLE MODIFICATION OF THE CLASSICAL BEUTLER TEST", JOURNAL OF INHERITED METABOLIC DISEASE, KLUWER, DORDRECHT, NL, vol. 35, no. Suppl.1, 1 September 2012 (2012-09-01), page S156, XP009184086, ISSN: 0141-8955
 • PEREZ BELEN ET AL: "A novel congenital disorder of glycosylation type without central nervous system involvement caused by mutations in the phosphoglucomutase 1 gene", JOURNAL OF INHERITED METABOLIC DISEASE, KLUWER, DORDRECHT, NL, vol. 36, no. 3, 1 May 2013 (2013-05-01), pages 535-542, XP009184087, ISSN: 0141-8955
 • MORAVA-KOZICZ E ET AL: "RECURRENT HYPOGLYCEMIAS: DEFINING A NOVEL, TREATABLE INBORN ERROR WITH PHOSPHOGLUCOMUTASE 1 DEFICIENCY", JOURNAL OF INVESTIGATIVE MEDICINE, LIPPINCOTT WILLIAMS & WILKINS, US, vol. 61, no. 2, 1 February 2013 (2013-02-01), page 494, XP009184088, ISSN: 1081-5589

• LAURA C. TEGTMEYER ET AL: "Multiple Phenotypes in Phosphoglucomutase 1 Deficiency", NEW ENGLAND JOURNAL OF MEDICINE, vol. 370, no. 6, 6 February 2014 (2014-02-06), pages 533-542, XP55187141, ISSN: 0028-4793, DOI: 10.1056/NEJMoa1206605

SEQUENCE LISTING

<110> Westfaelische Wilhelms-Universitaet Muenster

<120> Means and methods for diagnosing and treating CDG caused by a deficiency of PGM1

<130> UKM14982EP

<160> 41

<170> PatentIn version 3.5

<210> 1
<211> 6
<212> PRT
<213> Artificial Sequence

<220>
<223> Mg2+-binding site of PMG1

<400> 1

```
Asp Gly Asp Gly Asp Arg
1               5
```

<210> 2
<211> 7
<212> PRT
<213> Artificial Sequence

<220>
<223> sugar body binding site of PMG1

<400> 2

```
Cys Gly Glu Glu Ser Phe Gly
1               5
```

<210> 3
<211> 15
<212> PRT
<213> Artificial Sequence

<220>
<223> binding of the phosphate of the incoming phosphosugar of PMG1

<400> 3

```
Arg Leu Ser Gly Thr Gly Ser Ala Gly Ala Thr Ile Arg Leu Tyr
1               5               10                  15
```

<210> 4
<211> 4
<212> PRT
<213> Artificial Sequence

<220>
<223> region binding of PGM1 to Zasp protein

<400> 4

```
                                        Glx Ala Ser Pro
                                        1
```

<210> 5
<211> 12
<212> DNA
<213> Artificial Sequence

<220>
<223> c.1145-343

<400> 5
ttctgcaggc tg          12

<210> 6
<211> 12
<212> DNA
<213> Artificial Sequence

<220>
<223> c.1145-224

<400> 6
gagagtgaag gg          12

<210> 7
<211> 12
<212> DNA
<213> Artificial Sequence

<220>
<223> mRNA

<400> 7
gtccctccca ca          12

<210> 8
<211> 12
<212> DNA
<213> Artificial Sequence

<220>
<223> mRNA

<400> 8
tcactgctcc ag          12

<210> 9
<211> 6
<212> PRT
<213> Artificial Sequence

<220>

<223> Mg2+-binding site of PMG1

<400> 9

```
 Asp Gly Asp Gly Asp Arg
 1                   5
```

<210> 10
<211> 23
<212> DNA
<213> Artificial Sequence

<220>
<223> primer PGM1

<400> 10
aaagatggac tgtgggctgt cct        23

<210> 11
<211> 23
<212> DNA
<213> Artificial Sequence

<220>
<223> primer PGM1

<400> 11
cctcagcttc cacctcctcg taa        23

<210> 12
<211> 18
<212> DNA
<213> Artificial Sequence

<220>
<223> universal primer

<400> 12
tgtaaaacga cggccagt        18

<210> 13
<211> 18
<212> DNA
<213> Artificial Sequence

<220>
<223> universal primer

<400> 13
caggaaacag ctatgacc        18

<210> 14
<211> 20
<212> DNA
<213> Artificial Sequence

<220>

<223> Ex1 variant 1 - F primer

<400> 14
gtccctttaa ggaggagggc          20

<210> 15
<211> 20
<212> DNA
<213> Artificial Sequence

<220>
<223> Ex 1 variant - R primer

<400> 15
gagtcaggcg agcaggtctg          20

<210> 16
<211> 20
<212> DNA
<213> Artificial Sequence

<220>
<223> Ex1 variant 2 - F primer

<400> 16
ctttgtgtgt gggtgcgagt          20

<210> 17
<211> 20
<212> DNA
<213> Artificial Sequence

<220>
<223> Ex1 variant 2 - R primer

<400> 17
cttaagaccg ggagtgtgct          20

<210> 18
<211> 17
<212> DNA
<213> Artificial Sequence

<220>
<223> Ex 2 - F primer

<400> 18
ccttacagtg cacacag          17

<210> 19
<211> 20
<212> DNA
<213> Artificial Sequence

<220>
<223> Ex 2 - R primer

<400> 19

gaagcaaaga gcacctccct        20

<210> 20
<211> 17
<212> DNA
<213> Artificial Sequence

<220>
<223> Ex 3 - F primer

<400> 20
ccatgcgcaa atcttcc        17

<210> 21
<211> 20
<212> DNA
<213> Artificial Sequence

<220>
<223> Ex 3 - R primer

<400> 21
tcatgcagct gatccctggc        20

<210> 22
<211> 20
<212> DNA
<213> Artificial Sequence

<220>
<223> Ex 4 - F primer

<400> 22
atcccctgtg tgcctcatcc        20

<210> 23
<211> 20
<212> DNA
<213> Artificial Sequence

<220>
<223> Ex 4 - R primer

<400> 23
cttaccacga ggagtgtctc        20

<210> 24
<211> 20
<212> DNA
<213> Artificial Sequence

<220>
<223> Ex 5 - F primer

<400> 24
ctccactagt ccctgaacac        20

<210> 25

<211> 20
<212> DNA
<213> Artificial Sequence

<220>
<223> Ex 5 - R primer

<400> 25
gcatctagaa ctcacccagc        20

<210> 26
<211> 20
<212> DNA
<213> Artificial Sequence

<220>
<223> Ex 6 - F primer

<400> 26
gccttcggcc caatggtact        20

<210> 27
<211> 20
<212> DNA
<213> Artificial Sequence

<220>
<223> Ex 6 - R primer

<400> 27
gagaggggca ttgaatccag        20

<210> 28
<211> 20
<212> DNA
<213> Artificial Sequence

<220>
<223> Ex 7 - F primer

<400> 28
ttcttcttcc gccttctctg        20

<210> 29
<211> 20
<212> DNA
<213> Artificial Sequence

<220>
<223> Ex 7 - R primer

<400> 29
tccctgcaga ctcaatgacc        20

<210> 30
<211> 20
<212> DNA
<213> Artificial Sequence

<220>
<223> Ex 8 - F primer

<400> 30
cagcaagggg gaactttgtc          20

<210> 31
<211> 20
<212> DNA
<213> Artificial Sequence

<220>
<223> Ex 8 - R primer

<400> 31
catgtccacc caatggacac          20

<210> 32
<211> 20
<212> DNA
<213> Artificial Sequence

<220>
<223> Ex9 - F primer

<400> 32
gttccatccc cagtgactga          20

<210> 33
<211> 20
<212> DNA
<213> Artificial Sequence

<220>
<223> Ex9 - R primer

<400> 33
gtgggacagc agaaaaccag          20

<210> 34
<211> 20
<212> DNA
<213> Artificial Sequence

<220>
<223> Ex10 - F primer

<400> 34
cacccagcat cactgaggtc          20

<210> 35
<211> 20
<212> DNA
<213> Artificial Sequence

<220>
<223> Ex10 - R primer

<400> 35
caggcttgac ctcctgggct          20


<210> 36
<211> 20
<212> DNA
<213> Artificial Sequence


<220>
<223> Ex11 - F primer


<400> 36
gcagaggaag atgtcaggag          20


<210> 37
<211> 20
<212> DNA
<213> Artificial Sequence


<220>
<223> Ex11 - R primer


<400> 37
cagctcccaa gggcatcttg          20


<210> 38
<211> 20
<212> DNA
<213> Artificial Sequence


<220>
<223> I - F primer


<400> 38
cctttcccct cccgccggac          20


<210> 39
<211> 20
<212> DNA
<213> Artificial Sequence


<220>
<223> I - R primer


<400> 39
gcaccgagtt cttcacagag          20


<210> 40
<211> 20
<212> DNA
<213> Artificial Sequence


<220>
<223> II - F primer


<400> 40
ctgggccaaa ccgactgaag          20

<210> 41
<211> 18
<212> DNA
<213> Artificial Sequence

<220>
<223> II - R primer

<400> 41
tggatgggtc cgggggtg        18

## Description

## BACKGROUND

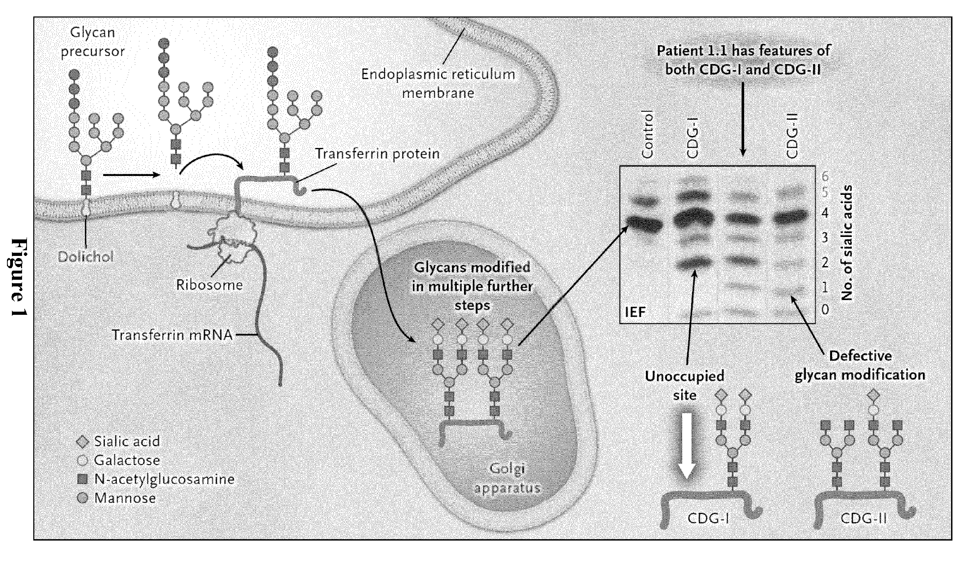

Protein N-glycosylation is a ubiquitous process in all organ systems. During N-glycosylation, glycan precursors are assembled from monosaccharide units and then covalently attached to asparagine residues in the nascent peptide chain of a protein (Fig. 1). The protein-bound glycans undergo further processing to generate mature glycoproteins. Genetic defects in protein N-glycosylation, designated as congenital disorders of glycosylation, lead to multisystem disorders. Mutations of genes involved in N-glycosylation may affect either the biosynthesis of the glycan precursor (congenital disorder of glycosylation type I [CDG-I]) or the processing of the glycan after its attachment to the protein (congenital disorder of glycosylation type II [CDG-II]) (Fig. 1).

Congenital disorders of glycosylation (CDG) are genetic syndromes that result in impaired glycoprotein production. CDGs are due to incomplete glycan attachments in glycoproteins. Since about 50% of all proteins are glycoproteins that may be affected by CDGs, typically systemic effects and a broad phenotypic spectrum are observed.

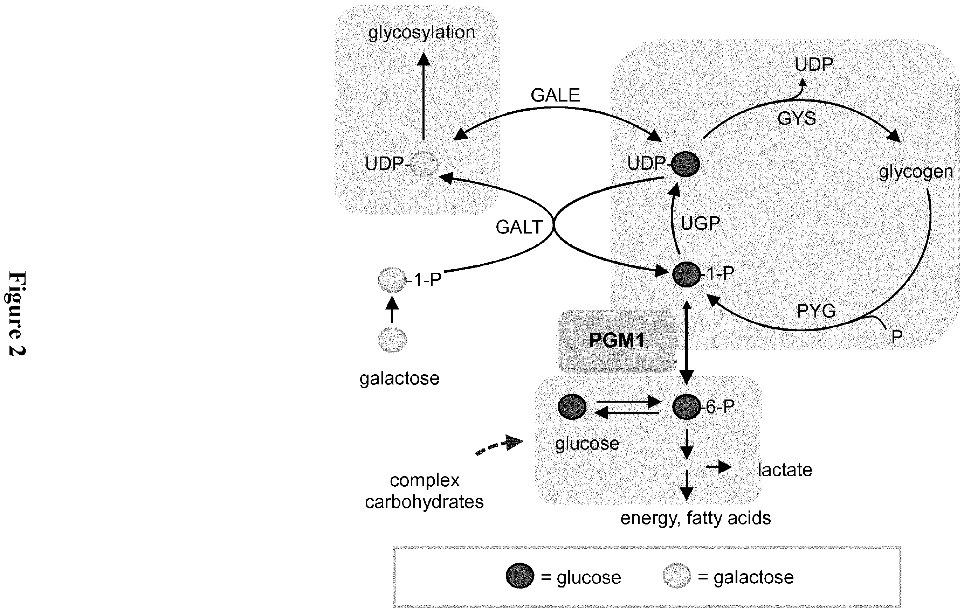

Glucose-1-phosphate is an important intermediate in the pathways leading to protein N-glycosylation and in glucose homeostasis. Phosphoglucomutase 1 (PGM1) catalyzes the interconversion of glucose-6-phosphate and glucose-1-phosphate (Fig. 2).

In 1966, Beutler and Baluda reported a spot screening test for GALT deficiency for the detection of glactosemia. The incidence of galactosemia is about 1 in 80,000 births in the U.S. If the disease is detected in the first few days of life, a newborn can be placed on special galactose-free diets to prevent the severe symptoms of the disease. Galactose is generally derived from lactose, which is the main carbohydrate in milk. When galactosemia is not detected in the first few days of life, it may cause convulsions, irritability, lethargy, poor feeding, poor weight gain, yellow skin and whites of the eyes or vomiting.

There are three enzymes whose deficiency leads to galactosemia: GALT, galactokinase, and UDPG galactose-4-epimerase. Classic galactosemia (GALT deficiency) is the most common. GALT deficiency is an inborn error of metabolism transmitted through an autosomal recessive gene. Untreated galactokinase-deficient patients suffer from cataracts, but other debilitating symptoms do not occur. The epimermase deficiency is quite rare and, in most cases, there are few clinical symptoms associated with epimerase deficiency. For a review of screening and diagnosis of galactosemia, see Beutler (1991).

In the reported spot screening test for GALT deficiency by Beutler and Baluda, NADP is reduced to NADPH as a result of a series of enzymatic reactions. The assay is very simple. Sample is mixed with reagent and allowed to incubate for a certain period of time. Drops of the reaction mixture are removed and spotted onto filter paper. NADPH fluorescence is then detected using a black light (long-wave UV light). If GALT activity is not present in a blood sample, NADP is not reduced to NADPH and no fluorescence results. In this assay, GALT catalyzes the reaction:

$$\text{GAL-1-P} + \text{UDPG} \xrightarrow{\text{GALT}} \text{G-1-P} + \text{UDP-GAL}$$

The above reaction is coupled to the following enzyme system to yield a fluorescent NADPH product:

$$\text{G-1-P} \xrightarrow{\text{PGM}} \text{G-6-P}$$

$$\text{G-6-P} + \text{NADP} \xrightarrow{\text{G-6-PDH}} \text{6-PGA} + \text{NADPH (fluorescent)}$$

$$\text{6-PGA} + \text{NADP} \xrightarrow{\text{6-PGD}} \text{R-5-P} + \text{NADPH (fluorescent)}$$

In the Beutler and Baluda procedure, a reaction mixture containing GAL-1-P, UDPG, and NADP is mixed with a blood sample and incubated at 37 °C. Since GALT is located in the red blood cell, whole blood or a dried blood spot is generally the sample of choice. Drops from the reaction mixture are spotted onto filter paper at time zero and at various intervals during the incubation. The spots are then visualized under UV light. Fluorescence indicates the presence of GALT activity. The fluorescence will range from bright for normal blood to no fluorescence for GALT deficient samples. If the blood is from a heterozygote for GALT deficiency, a subdued fluorescence results. Three enzymes required for the reaction are provided by the patient's blood: PGM, G-6-PDH (Glucose-6-phosphate dehydrogenase) and 6-phosphogluconate dehydrogenase (6-PGD(H)). This paper-spot assay has been commercialized by Sigma Diagnostics, St. Louis, Mo. (Procedure No. 195).

Methods which measure the NADPH product optically have also been reported. A kinetic micro-spectrophotometric assay of whole blood was done in an LKB Reaction Rate Analyzer (Pesce et al. 1977). In this assay a modified Beutler reagent is mixed with whole blood and the rate of NADPH formation is measured spectrophotometrically. Actual enzyme units can be calculated after hemoglobin concentration measurement.

The GALT assay has also been used to confirm the results of total (GAL plus GAL-1-P) galactose assays (Greenberg et al. 1989).

The use of microtiter plates and fluorescent readout has been recently described (Yamaguchi et al. 1989) in which total galactose (GAL plus GAL-1-P) was measured in blood dried on filter paper. A punched-out spot was first extracted with a methanol:acetone:water mixture, during which hemoglobin and other proteins were denatured. Water was then added and the extract transferred to an assay plate where a galactose dehydrogenase enzyme reagent converted galactose and NAD to products. The NADH product concentration, which is proportional to the original galactose concentration in the sample, was measured fluorometrically.

A manual fluorometric assay for GALT activity has also been reported (Frazier, Clemons, and Kirkman 1992). A dried blood spot on filter paper was mixed with a modified Beutler reagent and incubated for various amounts of time; after which a portion of the sample-reagent mixture was transferred to a holding reagent, which stopped the reaction. A Turner fluorometer was used to measure fluorescence.

This fluorescent spot test, however, is not a specific assay for GALT, but an assay cascade measuring consecutively 4 enzymes, namely GALT, PGM, G6PDH and 6-PGHD. Thus, samples that only show a phosphoglucomutase-1 deficiency may be misinterpreted as being a galactosemia, rather than congenital disorders of glycosylation (CDG) caused by deficiency of phosphoglucomutase-1.

With the foregoing background in mind, it is desirable to be able to perform an enzyme assay for only the activity of PGM-1, since also the therapy may be fairly different.

The technical problem underlying the present invention is therefore the provision of means and methods for diagnosing and/or treating diagnosing a mixed congenital disorder of glycosylation (CDG) caused by a deficiency of phosphoglucomutase-1 (PGM1). The solution to said problem is described herein and summarized in the claims, exemplified in the appended Examples and illustrated in the Figures.

The inventors performed homozygosity mapping followed by whole-exome sequencing was used to identify a mutation in the gene for phosphoglucomutase 1 (PGM1) in two siblings. Sequencing identified additional mutations in 15 other families. Phosphoglucomutase 1 enzyme activity was assayed on cell extracts. Analyses of glycosylation efficiency and quantitative studies of sugar metabolites were performed. Galactose supplementation in fibroblast cultures and dietary supplementation in the patients were studied to determine the effect on glycosylation.

The inventors found that phosphoglucomutase 1 enzyme activity was markedly diminished in all patients. Mass spectrometry of transferrin showed a loss of complete N-glycans and the presence of truncated glycans lacking galactose. Fibroblasts supplemented with galactose showed restoration of protein glycosylation and no evidence of glycogen accumulation. Dietary supplementation with galactose in six patients resulted in changes suggestive of clinical improvement. A new screening test showed good discrimination between patients and controls.

To this end, phosphoglucomutase 1 deficiency, previously identified as a glycogenosis, is also a congenital disorder of glycosylation. Supplementation with galactose leads to biochemical improvement in indexes of glycosylation in cells and patients, and supplementation with complex carbohydrates stabilizes blood glucose.

Having established the correlation between PGM1 and the CDG, the present inventors developed a modified version of the Beutler test, which is used with Guthrie heel-prick test cards (dried blood spots) to screen for congenital disorders of glycosylation (CDG) caused by deficiency of phosphoglucomutase-1, by exchanging the substrates for the GALT with the substrate for PGM or G6PDH to specifically detect congenital disorders of glycosylation (CDG) caused by deficiency of phosphoglucomutase-1. Specifically, phosphoglucomutase-1 is dissolved out a punched piece of the dried blot spot via hemolysis. The enzyme is then offered glucose-1-phosphate as substrate. This substrate can be converted to glucose-6-phosphate by phosphoglucomutase-1 activity. Then an indicator reaction can take place. For such an indicator reaction, NADPH+ is added and can be converted to its reduced form NADPH with two dehydrogenases present in the hemolysate. The so-developed NADPH fluoresces in long-wave UV light. The intensity of the fluorescence is proportional to the phosphoglucomutase-1 activity in the probe. The phosphoglucomutase-1 activity present in the probe can then be calculated based on a calibration curve.

## DESCRPTION

Accordingly, in a first aspect the present invention provides a method for diagnosing a mixed congenital disorder of glycosylation (CDG) caused by a deficiency of phosphoglucomutase-1 (PGM1) comprising

(a) adding separately (i) glucose-1-phosphate (G1P) and (ii) glucose-6-phosphate (G6P) in a Beutler test format to a sample obtained from a subject; and
(b) comparing the signal from (i) and (ii),
wherein the subject suffers from said CDG, if the signal (ii) exceeds the signal of (i).

The methods of the present invention can also be used for the detection of a genetic disorder of the PGM1 gene. A genetic disorder includes all genetic aberrations leading to PGM1-enzyme deficiency.

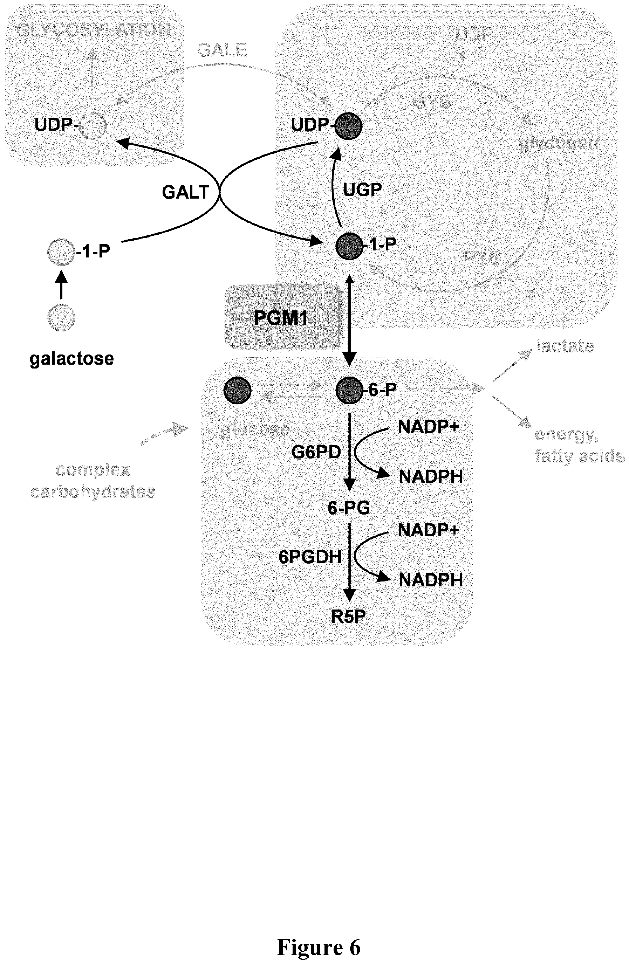

The Beutler test format is known in the art. When used herein, the term "Beutler test format" is not strictly limited to the Beutler test as such as published in 1966, but also includes the meanwhile established variants thereof, such as the Beutler test format disclosed in EP 0763132 or the Beutler test format as provided by Biorad or Roche. It is thus understood that the term "Beutler test format" describes in essence a test format which makes use of dried blood samples on a suitable support or carrier which is contacted with suitable substrates in order to detect the generation of NADPH in the glucose/galactose pathway as depicted, for example, in Figure 6.

The term "adding" when used in the methods described herein includes that a sample obtained from a patient is contacted or brought into contact with an exogenously added substance described herein, for example, glucose-1-phosphate, glucose-6-phosphate, glucose-1,6-bisphosphate, and/or PMG1.

The term "separately" when used in the context of the methods and means of the present invention means that glucose-1-phosphate (G1P) and (ii) glucose-6-phosphate (G6P) are added independently from each other to a sample obtained from a subject. Preferably, glucose-1-phosphate (G1P) and (ii) glucose-6-phosphate (G6P) are each added to a sample from the same subject, i.e., G1P is added to a first sample and G6P is added to a second sample, whereby the first and second sample is from the same subject. However, if added to one sample, preferably G1P is added first, followed by G6P.

The mixed congenital disorder of glycosylation (CDG) caused by a deficiency of phosphoglucomutase-1 (PGM1) is also referred to herein as PGM1-CDG.

A subject when used herein is preferably a newborn.

A signal when referred to herein is preferably fluorescence.

A sample when used herein can be any sample from a subject, such as a muscle cell. Preferably, the sample is a blood sample, preferably a red blood sample. As used herein, the term "red blood sample" shall be construed to include any hemoglobin-containing blood or blood product, such as whole blood, washed red blood cells, a hemolysate made from red blood cells, and a dried whole blood sample on a physical support, the latter of which is preferred.

It is a preferred embodiment of the means and methods of the present invention that PGM1 is further/additionally added. The addition of PGM1 serves as a control in that it shows that the Beutler test format is working, since PGM1 catalyzes the reaction from G1P to G6P which then results in NADPH formation which can be visualized in the form of a signal, preferably fluorescence signal. Similarly, in another preferred embodiment, Glucose-6-phosphate-Dehydrogenase (G6PD), and/or 6-Phosphogluconat-dehydrogenase (6PGDH) can be further/additionally added.

In another preferred embodiment of the means and methods of the present invention, glucose-1,6-bisphosphate is present in the Beutler test format. The inventors, without being bound by theory, found that glucose-1,6-bisphosphate aids in the diagnosis as described herein. Glucose-1,6-bisphosphate is preferably present in the range between about 5 μM to about 200 μM.

In a further aspect, the present invention provides for glucose-1-phospate for use in a method of diagnosing a mixed congenital disorder of glycosylation (CDG) caused by a deficiency of phosphoglucomutase-1 (PGM1).

In a still further aspect, the present invention provides for Glucose-6-phospate for use in a method of diagnosing a mixed congenital disorder of glycosylation (CDG) caused by a deficiency of phosphoglucomutase-1 (PGM1).

In another aspect, the present invention provides for Glucose-1-phospate and Glucose-6-phospate for use in a method of diagnosing a mixed congenital disorder of glycosylation (CDG) caused by a deficiency of phosphoglucomutase-1 (PGM1).

Also, the present invention provides for a method for diagnosing a mixed congenital disorder of glycosylation (CDG) caused by a deficiency of phosphoglucomutase-1 (PGM1) comprising subjecting a sample obtained from a subject to a Beutler test in the (i) absence and (ii) presence of exogenously added PGM1, wherein the subject suffers from said CDG, if the signal (ii) exceeds the signal of (i).

Furthermore, the present invention provides for PGM1 for use in a method of diagnosing a mixed CDG caused by a deficiency of PGM1, said method comprising subjecting a sample obtained from a subject to a Beutler test in the (i) absence and (ii) presence of exogenously added PGM1, wherein the subject suffers from said CDG, if the signal (ii) exceeds the signal of (i).

In a still further aspect, the present invention provides a kit comprising a Beutler assay format, said Beutler assay format comprising glucose-1-phosphate and/or glucose-6-phosphate and/or glucose-1,6-bisphosphate.

Preferably, in the kit glucose-1-phosphate and/or glucose-6-phosphate and/or glucose-1,6-bisphosphate is coated onto a solid support.

In yet another aspect, the present invention relates to a Beutler assay format comprising glucose-1-phosphate and/or glucose-6-phosphate and/or glucose-1,6-bisphosphate. Preferably, in the Beutler assay format, said glucose-1-phosphate and/or glucose-6-phosphate and/or glucose-1,6-bisphosphate is coated onto a solid support.

A still further aspect of the present invention is a method of treating a mixed CDG caused by a deficiency of PGM1 in a subject in need thereof, said method comprising administering a therapeutically effective amount of galactose to said subject in need thereof. The subject that is treated is preferably identified by the method of diagnosis of the present invention, or by applying the kit or the Beutler assay format as described herein.

In another aspect, the present invention relates to galactose for use in a method of treating a mixed CDG caused by a deficiency of PGM1 in a subject. The subject that is treated is preferably identified by the method of diagnosis of the present invention, or by applying the kit or the Beutler assay format as described herein.

Preferably, a subject is administered galactose orally, preferably in the range of about 0.1 - 5.0 g per kg b.w., more preferably in the range of 0.1 to 1.0 g per kg b.w., most preferably in the range of 0.1 to 0.5 g per kg b.w.

## FIGURES

**Figure 1. Glycoprotein Biosynthesis and Congenital Disorders of Glycosylation.**

For the N-glycosylation of proteins, a glycan precursor is assembled from monosaccharide units on an anchoring isoprenoid alcohol, dolichol, in the membrane of the endoplasmic reticulum. Each glycan precursor is then transferred en bloc to a specific asparagine residue in the nascent peptide chain of a protein being synthesized by a ribosome and entering the endoplasmic reticulum. Because transferrin (brown line) is abundant in blood, it is used as an indicator of the efficiency of the glycosylation system. Transferrin has two glycosylation sites. The protein is transported to the Golgi apparatus, and the glycans are modified in multiple steps. Each fully modified glycan is terminated with two negatively charged sialic acid residues. Thus, the transferrin molecule carries a total of four sialic acid residues. If a genetic defect impairs synthesis of the precursor glycan in the endoplasmic reticulum, one or both glycosylation sites of transferrin may remain unoccupied (white arrow). Such defects are classified as congenital disorders of glycosylation type I (CDG-I). In congenital disorders of glycosylation type II (CDG-II), the defect occurs in the modification of the already-transferred glycan. Both glycosylation residues may carry a glycan, but those glycans may be missing terminal sialic acid residues and end with galactose, mannose, or N-acetylglucosamine. Isoelectric focusing (IEF), which separates molecules according to differences in their isoelectric point, is used for investigating congenital disorders of glycosylation with the use of an agarose-gel-based system, followed by immunoprecipitation in gel with an antibody to transferrin and final staining of the precipitate with Coomassie blue. Under conditions of normal glycosylation, most transferrin molecules carry four sialic acid residues (tetrasialo-transferrin) and form a single major band on IEF. A few transferrin molecules carry five or six sialic acid residues that appear as minor bands on IEF (gray numbers; not shown in the molecular-structure diagrams). CDG-I defects are easily identified owing to the occurrence of transferrin isoforms with two or no sialic acid residues detected by means of IEF. No signal for monosialotransferrin (transferrin with one sialic acid residue) is seen in such disorders. In CDG-II, the defect can result in transferrin molecules with no, one, two, three, or four sialic acid residues. A patient with a CDG-II defect typically has bands of approximately equal intensity at all positions from 0 through 3 or a smooth gradient of intensities, whereas in most cases, tetrasialotransferrin shows a more intense signal owing to residual activity. In our patients, the signal of transferrin with two sialic acid residues (disialotransferrin) may be more intense than those for either one or three residues (as in the sample from Patient 1.1). The signal with two sialic acid residues presumably arises from a mixture of transferrin molecules that have one unoccupied glycosylation site as well as transferrin molecules that are occupied at both sites by glycans but have only two terminal sialic acid residues. The patients in this study had features of both CDG-I and CDG-II, as shown by IEF, suggesting a novel disorder.

**Figure 2. Role of Phosphoglucomutase 1 in Sugar Metabolism.**

When blood glucose is low, glycogen degradation (yellow box) generates glucose-1-phosphate, which is converted by phosphoglucomutase 1 (PGM1) to glucose-6-phosphate for glucose release from the liver or subsequent catabolism in other tissues. In the reverse reaction, glucose-6-phosphate is converted to glucose-1-phosphate by means of PGM1 and is used as a substrate in the production of uridine diphosphate (UDP)-glucose, which can be used for the synthesis of glycogen or for protein glycosylation. Dietary galactose may be converted to UDP-galactose by galactose-1-phosphate uridyl-transferase (GALT) and used for protein glycosylation. UDP-galactose may also be converted into UDP-glucose by UDP-galactose epimerase (GALE). The GALT reaction is reversible; for clarity, only one direction of the coupled GALT reactions is shown. UGP denotes UDP-glucose pyrophosphorylase.

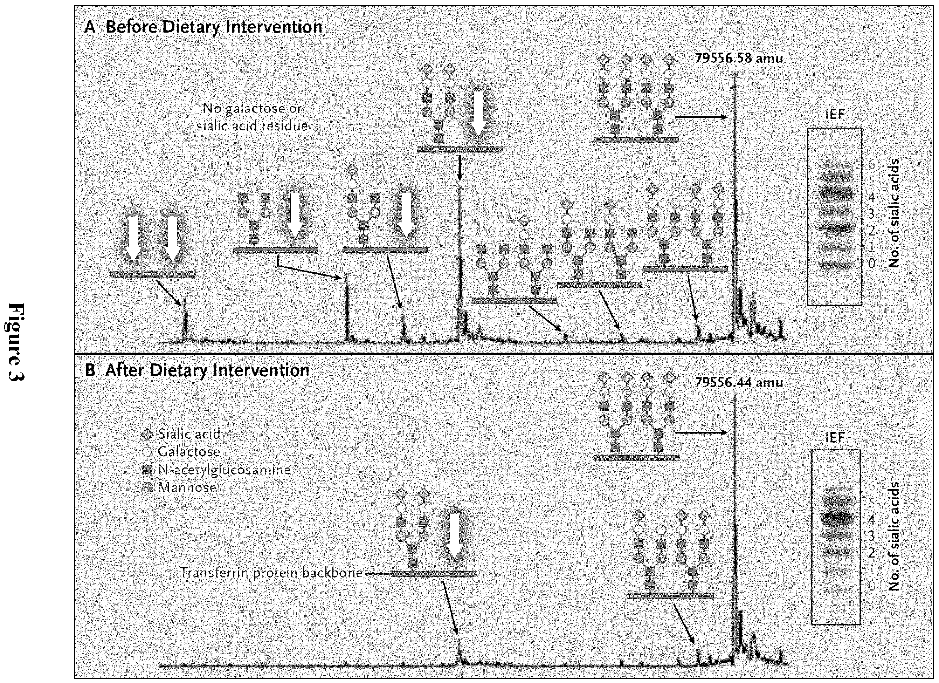

**Figure 3. Effects of Dietary Galactose on Protein Glycosylation.**

The results of mass spectrometry of the intact transferrin protein and corresponding transferrin IEF profiles are shown before (Panel A) and after (Panel B) the intake of supplementary galactose. The glycan structures are shown in detail with the transferrin protein backbone (brown line). Patients with PGM1 deficiency have highly specific transferrin glycosylation profiles. Data from Patient 2 show several peaks indicating the presence of glycoforms completely lacking one or both N-glycans (white arrows), a finding that is also observed in CDG-I. There are also peaks indicating the presence of glycoforms with truncated glycans lacking galactose (ending with N-acetylglucosamine; yellow arrows). Several structures end this way, which is typically seen in CDG-II. Numbers at main peaks are deconvoluted mass (amu). Numbers next to the IEF results indicate individual bands corresponding to numbers of sialic acid residues (black numbers indicate structures shown along the respective mass spectrum, and gray numbers structures not shown). After the patient increased dietary intake of galactose, protein glycosylation was substantially improved (Panel B). Peaks indicating the presence of glycoforms with truncated glycans lacking galactose almost completely disappeared, and peaks indicating the presence of glycoforms with complete loss of one or both N-glycans were considerably reduced. For details on the method and quantification of isoforms by means of liquid chromatography-mass spectrometry, see the Examples II.

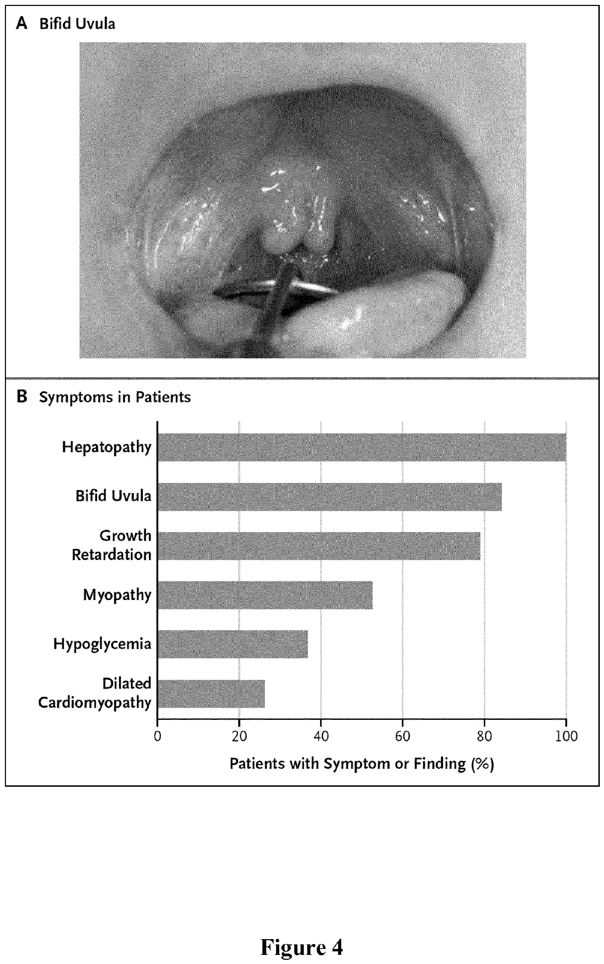

**Figure 4. Overview of Clinical Features of PGM1 Deficiency.**

Most patients had a bifid uvula (Panel A). Symptoms or findings related to PGM1 deficiency are listed according to frequency (Panel B). Hepatopathy was defined as elevated aminotransferase levels, steatosis, fibrosis, or a combination of these features. Myopathy was defined as a maximal creatine kinase level of more than 300 U per liter. Growth retardation was defined as a height at or below the 5th percentile. Hypoglycemia was defined as a fasting glucose level of less than 2.2 mmol per liter (40 mg per deciliter).

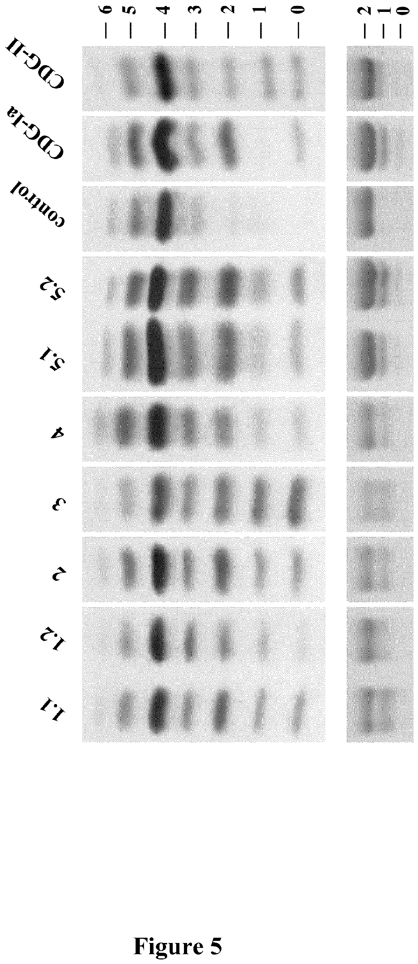

**Figure 5. Isoelectric Focusing and SDS-PAGE of Transferrin.**

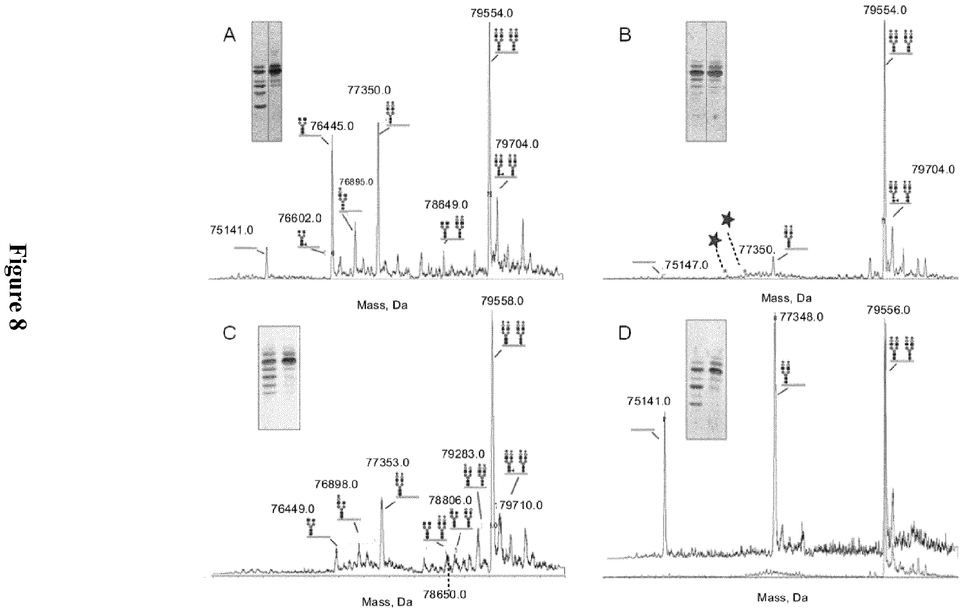

In most patients, IEF showed a CDG-II like pattern with increased amounts of all hyposialylated transferrin isoforms and a decreased amount of the fully glycosylated tetrasialo-transferrin (top panel, number of sialic acids at right side, compare Fig. 1). SDS-PAGE (lower panel) provides additional information: Transferrin with both glycosylation sites occupied by a glycan was the major signal in most patients (position label "2" at right side: number of glycosylation sites occupied by a glycan). A molecular weight loss of 2 kDa corresponding to the complete lack of one oligosaccharide side chain suggested also a CDG-I-component (position label "1"). Also visible: loss of 4 kDa due to both glycosylation sites unoccupied in CDG-Ia positive control lane (position label "0"). Since transferrin with 1 sialic acid (position labeled 1 at top panel) is due to CDG-II defect, i.e. incomplete modification of already transferred glycan chain, patients have a mixed type of CDG-I and -II. Multiple smaller fuzzy bands in the lower panel represent truncations of the side chains. Individuals 1.1 and 1.2 as well as 5.1 and 5.2 are sibs indicating that variability of transferrin glycosylation may occur within sib pairs carrying the same mutations: 1.2 and 5.1 show a relatively less intense CDG-I-component in IEF (signals at 2 and 0) and SDS (signals at 1 and 0) than their respective sibs. Additional IEF patterns of patient 6 and 8 are shown in Fig. 8. Patient numbers are used as in Table S1.

**Figure 6. The Beutler Test and the Modified Beutler Test.**

In this figure, the metabolic pathways for glucose and galactose that are of direct relevance to phosphoglucomutase 1 (PGM1) deficiency are shown as in Figure 2, but parts not relevant for the test are dimmed. However, the further metabolic pathway down to ribulose-5-phosphate (R5P) that is also relevant for the Beutler test and the modified Beutler test is now also shown. In the Beutler test for galactosemia, the substrates galactose-1-phosphate and UDP-glucose are included in the test reagent and used to assay Guthrie heel prick test cards spotted with the patient's blood sample. In the presence of GALT, these substrates generate glucose-1-phosphate, which is then converted to glucose-6-phosphate by PGM1, then to 6-phosphogluconate by G6PD (glucose-6-phosphate dehydrogenase) and then to ribulose-5-phosphate by 6PGDH (6-phosphogluconate dehydrogenase). Each of these last two reactions is coupled to the conversion of NADP+ to NADPH. The fluorescence of NADPH is used as the basis for the Beutler test assay. Thus the Beutler test requires the presence of several functional enzymes including GALT, PGM1, G6PD, and 6PGDH (although in general the rate-limiting enzyme is GALT). In a modification of the Beutler test evaluated in this study, galactose-1-phosphate and UDP-glucose were replaced by glucose-1-phosphate as the assay substrate. A similar assay was also developed using glucose-6-phosphate as the assay substrate in order to bypass PGM1.

Keys: ● = glucose, ○ = galactose, PGM1 = phosphoglucomutase 1, GALT = galactose-1-phosphate uridyltransferase, GALE = UDP-galactose epimerase, UGP = UDP-glucose pyrophosphorylase, GYS = glycogen synthase, PYG = glycogen phosphorylase, G6PD = glucose-6-phosphate dehydrogenase, 6-PG = 6-phosphogluconate, 6PGDH = 6-phosphogluconate dehydrogenase, R5P = ribulose-5-phosphate.

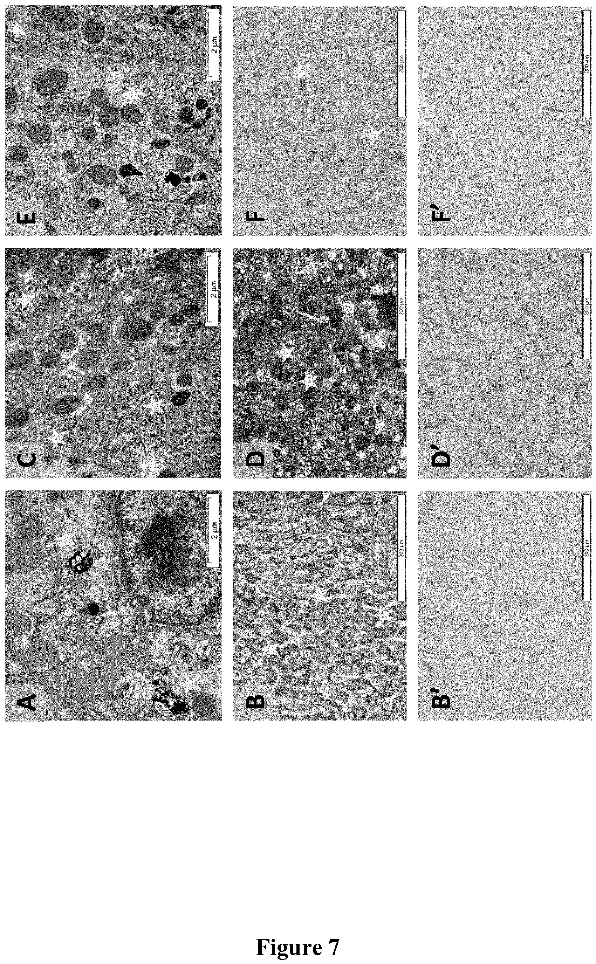

**Figure 7. Glycogen in Hepatocytes by Electron Microscopy and Light Microscopy.**

Liver tissue sections for electron microscopy (EM) were obtained from samples fixed in 2.5% glutaraldehyde in 0.1 M cacodylate buffer, post-fixed in buffered 2% osmium tetroxide, and processed to Epon 812. Sections routinely stained for EM with uranyl acetate and lead citrate were analyzed with a Jeol Jem 1011 transmission microscope equipped with digital camera. Tissue samples for light microscope (LM) were fixed in 4% buffered formalin and processed to paraffin blocks. De-paraffinized 5 μm thick control sections were diastase treated and stained by periodic acid-Schiff reaction (PAS). (A,B) PGM1-deficient hepatocytes (patient 6) showing glycogen particles (pale material, asterix) in the cytoplasm in EM (A), and under LM in PAS stained sections (B) less abundant than in glycogen storage disease la (GSD-la) (C,D) but more abundant than in controls with for example liver cholestasis (E,F). Glycogen in GSD-la is increased and stained darker than in PGM1-deficiency, in addition dark dots of glycogen aggregates are visible (C). (B',D',F') Lack of glycogen particles on sections diastase treated and PAS stained, respectively liver of patient with PGM1-deficiency, GSD-la and cholestasis.

**Figure 8. Variability of Transferrin Glycosylation, Details by Mass Spectrometry.**

While the CDG-Ia aberrant pattern in panel D is demonstrated clearly by the two structures showing loss of one or both complete glycans, the PGM1-deficient patients show a variable mix of those two structures and other peaks where the glycans are lacking galactose and sialic acid, i.e. they show a mixed type CDG-I/II. The degree of glycosylation deficiency is also highly variable.

Panels A, B and C show spectra of the three PGM1 patients 6, 8, and 1.2 respectively. Panel D shows an overlay of a control (red) and CDG-Ia patient (blue). Corresponding transferrin isoelectric focusing gels are shown in the first lane. In the second lane a control sample is depicted as reference (panels A-C). In panel D, the first lane corresponds to the blue tracing, the second lane with the red spectrum. The symbol "★" indicates the presence of truncated transferrin glycans for patient 8. Gray bars indicate the transferrin protein backbone.

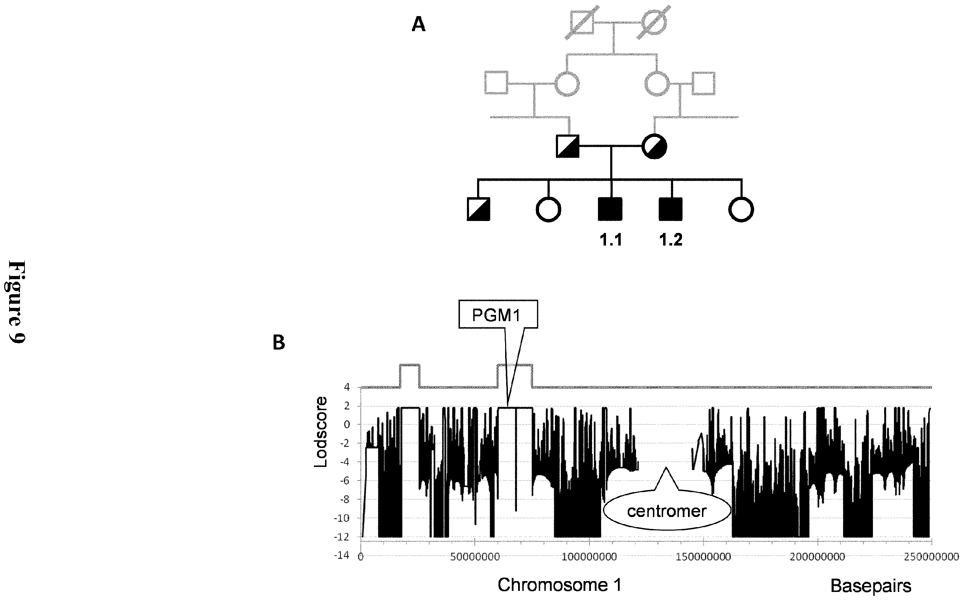

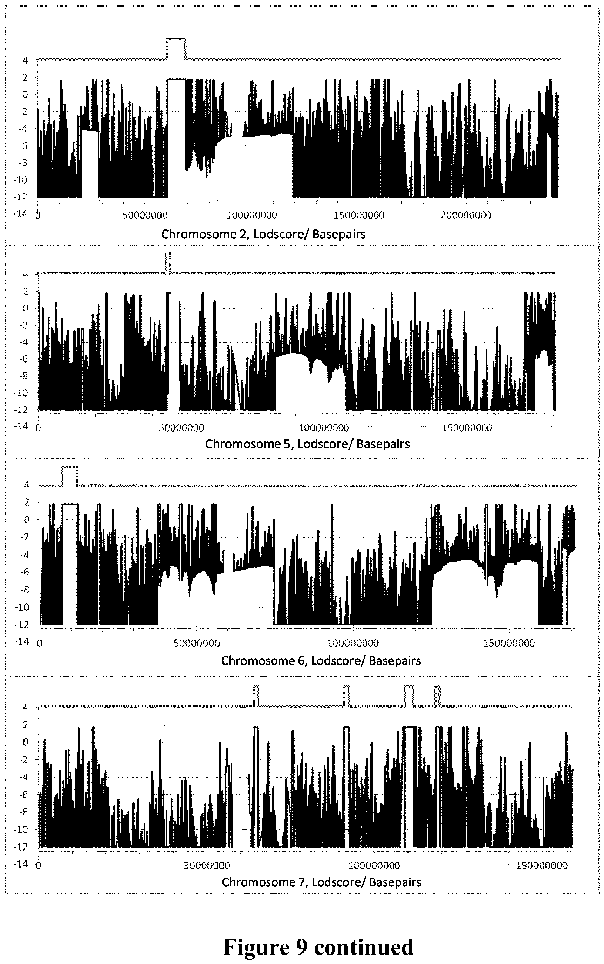

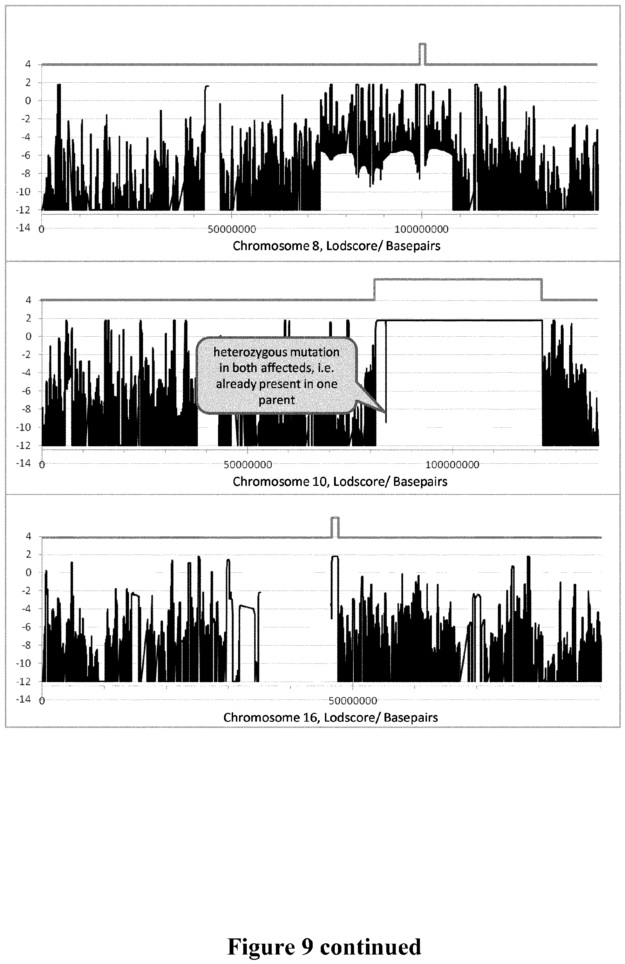

**Figure 9. Pedigree of Family 1 and Homozygosity Mapping.**

A. A male sib pair (individuals 1.1 and 1.2; Table S1) affected by the mixed type CDG-disease was used for definition of a candidate region by homozygosity mapping. Grey symbols: no DNA available. Black shading of symbols shows segregation of the mutation identified in PGM1: full filled symbols: homozygotes, half-filled symbols: heterozygotes.

B. Mapping result shown for chromosome 1. Details of the procedure are given in methods (Example II). The maximal lod score was 1.8057. Elevated green line indicates homozygous candidate regions.

C. Mapping results of all other autosomal chromosomes with homozygous regions > 1 Mb.

Mapping and next generation sequencing led to the identification of PGM1 as the primary candidate with a point mutation in this gene. The parents and additional unaffected sibs were used to demonstrate segregation of this mutation in the pedigree leading to disease in the homozygous mutation carriers only (black shading in panel A).

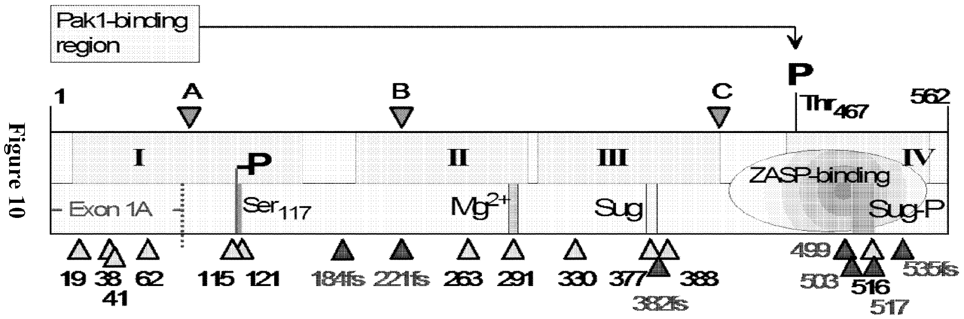

**Figure 10. Details on PGM1 Structure and Mutations.**

Schematic drawing of PGM1 with four domains (30,31) that are shown as grey shadowing (limits from Arimura (29)). Structural features as published are: PAK1 binding region < pos. 90 (32); catalytic site phospho-serine (33) (pos. 117, conserved amino acids 116-120; numbering based on UniProtKB/ Swiss-Prot:P00949); Mg2+-binding site (DGDGDR, pos. 288-93; SEQ ID NO: 1) (31); Sug (CGEESFG, pos. 374-80; SEQ ID NO: 2) (31): binding of the sugar body; Sug-P (RLSGTGSAGATIRLY, pos. 503-517; SEQ ID NO: 3): binding of the phosphate of the incoming phosphosugar (31); ZASP (pos. 427-562, most likely 489-562 (29) SEQ ID NO: 4): region binding of PGM1 to Zasp protein; Thr467; threonine phosphorylated by PAK1 leading to increased PGM1 activity. (32,34) Three common missense variants are indicated as A (rs855314, I88V), B (rs1126728, R221C), and C (rs11208257, Y420H) above the ribbon with green triangles. Variants B and C change the charge of the protein and explain the common IEF-variants of PGM1 (35), used for decades in paternity testing. PGM1-deficiency mutations are indicated below the ribbon with yellow (missense) or red (stop) triangles and the (first affected) amino acid position (details of mutations s. Table S1). In fast muscle a rare alternative transcript has been described using an alternative exon 1, so that all mutations of the regular exon 1A will be excluded.

Frameshift mutations F184Sfs*9, R221Vfs*13 are sufficiently described in Table S1. In cDNA of sibs 5.1/2, the mutation c.1145-222G>T creates a new splice donor site, causing use of a cryptic splice site 120 bp upstream as acceptor site. Thus, the mutated allele produces a mRNA containing an additional exon derived from intronic sequence (r.[1145-222g>u; 1144_1145ins1145-343_1145-224] with the additional sequence c.1145-343_1145-224: TTCTGCAGGCTG (SEQ ID NO: 5)... GAGAGTGAAGGG (SEQ ID NO: 6)). This translates to G382Vfs*23 at protein level. In patient 12, the mutation 1600-523G>A (M535Pfs*60) creates a new splice acceptor site and in addition downstream a cryptic splice donor site within the normal intron is activated. Thus, also in this case an additional exon is generated with insertion of 149 bases in the mRNA: r.[1600-523g>a;1599_1600ins1600-521_1600-373] (GTCCCTCCCACA (SEQ ID NO: 7)... TCACTGCTCCAG (SEQ ID NO: 8)). RNA of patient 8 for analysis of 1145-1G>C was not available.

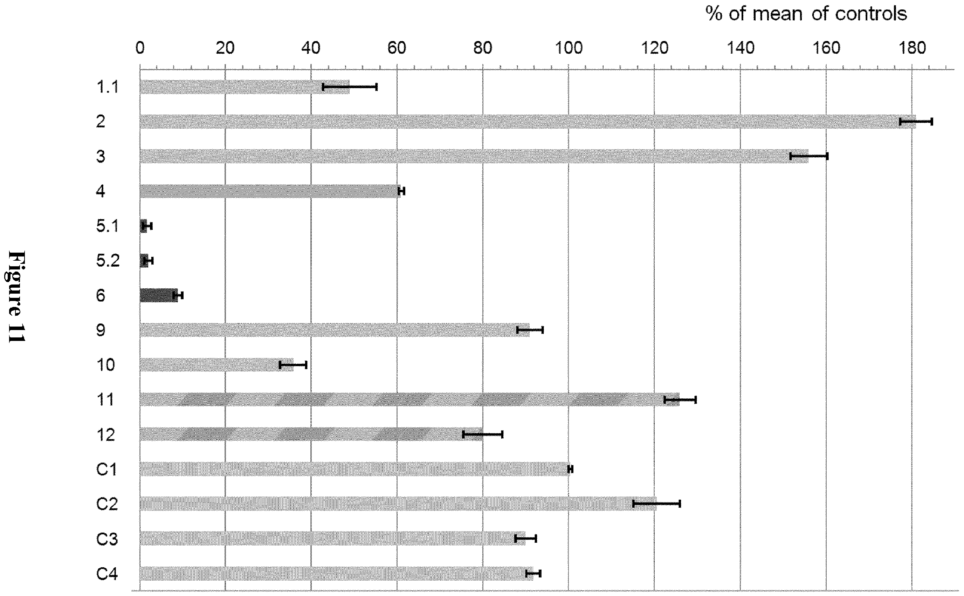

**Figure 11. Real-Time PCR for Quantification of PGM1-mRNA in Fibroblasts.**

We obtained either fibroblasts for cell culture, frozen fibroblast pellets for RNA-isolation, isolated RNA or cDNA from 11 patients and 4 controls. Numbers at the left are patient identifiers as in Table S1. C1-C4 are four different controls (grey bars). Results from patients with a homozygous stop codon leading to nonsense-mediated mRNA decay (36) are shown in red (5.1, 5.2, 6). The orange bar represents patient 4, a compound heterozygote having one nonsense allele expected to undergo nonsense-mediated decay. The light red/green hatching was used for compound heterozygotes (11, 12) having one nonsense allele, that is expected to be stable, since the position of the stop codon is predicted not to elicit nonsense-mediated mRNA-decay. (36) All other patients carrying missense variants are represented by green bars. To predict expected changes in mRNA-level from nonsense mutations, we determined the position of the different stop codons relative to the last splice junction. According to the rules and mechanisms reported in literature nonsense-mediated mRNA decay was expected only in homozygous patients 5.1, 5.2, and 6 as well as in the heterozygote 4, since only in these cases the stop codon was at least 50 bp before the last splice junction. (36) Note that mRNA of patient 6 showed some escape from nonsense-mediated decay (mRNA-level 8.9% of controls).

The method is described above, section "Real-time PCR for quantification of PGM1 mRNA" PGM1-mRNA levels show some variability that in part may be due to the various sources of cDNA analyzed. To check PGM1-levels in population we used data from a large scale study of mRNA-levels of about 39000 transcripts in 427 liver samples (37) that were available from Gene Expression Omnibus database entry GSE9588. Analysis demonstrated a calculated standard deviation of PGM1-levels of 54% but an asymmetric distribution with a range from 45 to 240% of mean PGM1-mRNA-level covering 95% of samples. In addition these authors reported an association of a frequent SNP in the 3'UTR of PGM1 (rs4643, minor allele frequency 20%) with PGM1 expression level (p = 1.01E-06). This SNP has also been reported to be associated with variability of the reduced glycosylation ("CDT", carbohydrate-deficient transferrin) that is observed as consequence of alcohol abuse.(38)

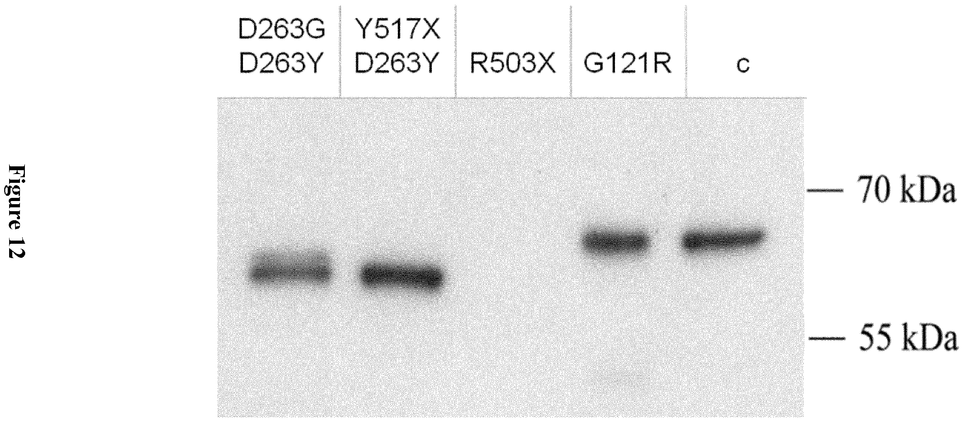

**Figure 12. Western Blot of PGM1 from PGM1 Deficient Fibroblasts.**

In 13 patients PGM1 protein was analyzed by Western blot. Often, no protein was visible as in the third lane (R530X-mutation, patient 6; for patient numbers see Table S1) or only very faint bands could be observed (not shown). The mutations D263Y and D263G show lower molecular masses (patient 3 in lane 1, patient 11 in lane 2). Analysis of the PGM1 transcript revealed no truncations so that the nature of the apparent mass loss remains unclear but may be partial degradation of the protein.

Normal amount of regular sized protein was observed for G121R (lane 4, patient 7) and G291R (patient 9, not shown). Nevertheless PGM1 activity was close to background for G121R - this is likely due to an effect on the catalytic active center, with Ser-117 carrying the phosphate-group involved in the phosphoglucomutase reaction. G291 (39) is located within a Mg2+ binding site indicated as blue bar in Fig. 10, see above, that in a 3-D-model of PGM1 is in close vicinity to the Ser-117-P and is involved in coordination of the phosphate and necessary for catalysis (DGDGDR, pos. 288-93; SEQ ID NO: 9). (31)

In summary, patients 3, 7, 9, and 11 showed some considerable protein signal, while 1.1, 1.2, 2, 4, 5.1, 5.2, 6, 10, 12 showed no or only very faint signal. From patients 8, 13.1/2, 14, 15, 16, carrying additional missense mutations T19A, D62H, T115A and E388K, no protein samples were available for analysis.

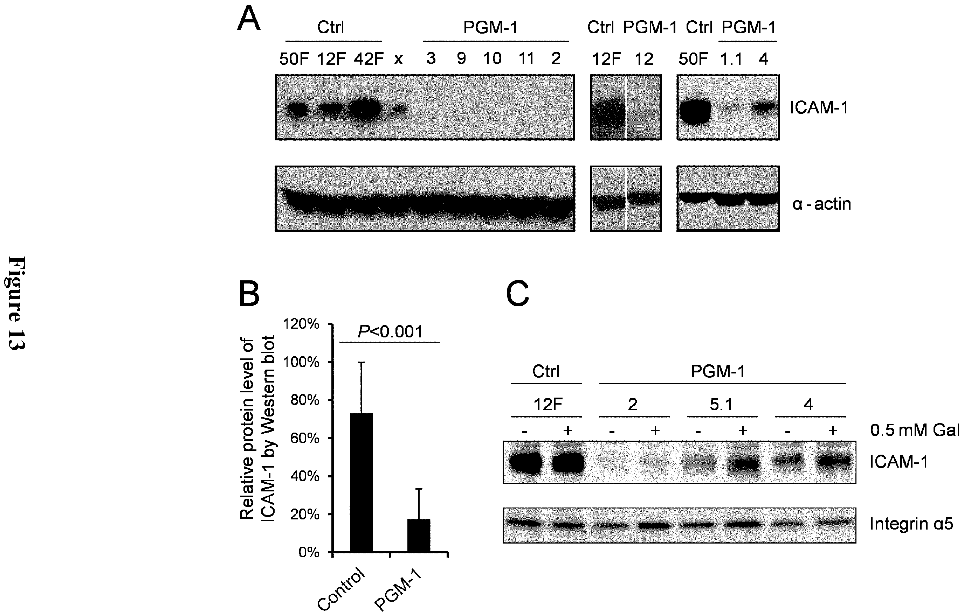

**Figure 13. Western Blotting of Fibroblast ICAM-1.**

ICAM-1 has 8 occupied N-glycosylation sites (40) and is highly suitable to check cultured cells for glycosylation efficiency, since ICAM-1 on Western blots is strongly diminished due to underglycosylation. (10) The figure demonstrates that ICAM-1 is a suitable measure for glycosylation efficiency also in PGM1-deficient cells (A and B). Galactose supplementation could partially restore the ICAM-1 expression in PGM-1 patients' cells (increase of 1.5-fold up to 8-fold, panel C). PGM-1 sample numbers correspond to those of Table S1.

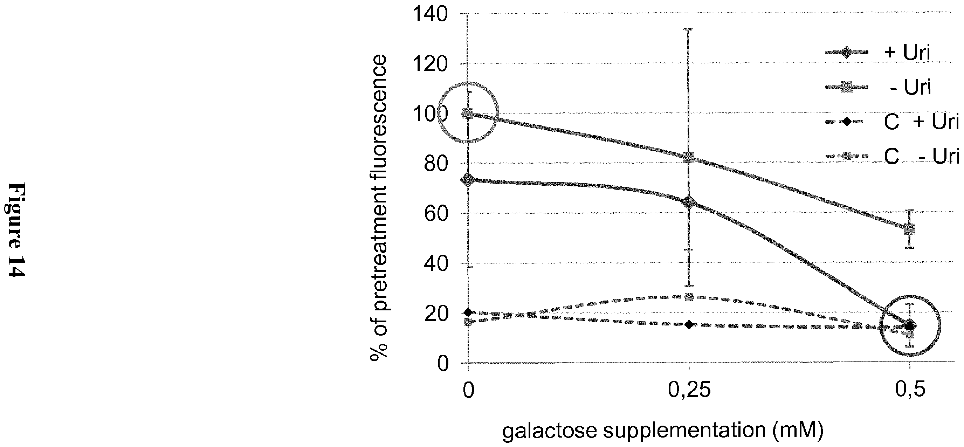

**Figure 14. Galactose and Uridine Supplementation in Patients' Fibroblasts**

ER-retained GFP, engineered to contain an N-glycosylation site that destroys fluorescence when it is glycosylated (Glyc-ER-GFP) (11), was transfected into PGM1-deficient fibroblasts as a marker for impaired glycosylation. Testing different conditions in cell culture (see examples II; method: Assay for cellular glycosylation efficiency using modified GFP) we found that supplementation of 3 different patient cell lines with 0.5mM galactose and 100µM uridine reduced fluorescence of Glyc-ER-GFP to 15% (+-7% (SD); red circle) of the individual pretreatment fluorescence (100%: blue circle). Normal control cells almost completely glycosylate the GFP and background fluorescence is typically about 10-20% of untreated values from patient cells (broken lines). This means that combined optimal supplementation with galactose and uridine achieved fully normal glycosylation in patient cells - see overlay of controls and patient data points in the red circle.

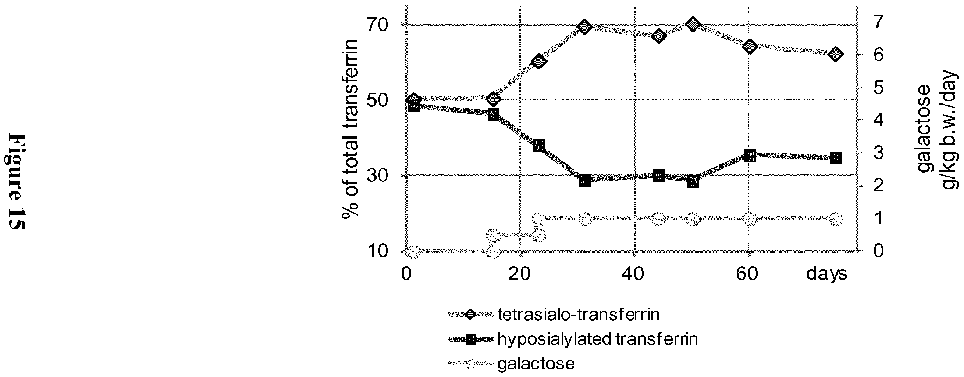

**Figure 15. Dietary Effect of Galactose on Transferrin Glycosylation**

Patient 1.1 started to take galactose at a daily dose of 0.5 g per kg b.w. divided into 3 portions at day 15, doubled after one week. Effect on transferrin isoforms was quantitated by HPLC (tetrasialo and hyposialylated forms). Within a few weeks tetrasialo-transferrin had increased from 50 up to 70% with a corresponding decrease of the hyposialylated isoforms.

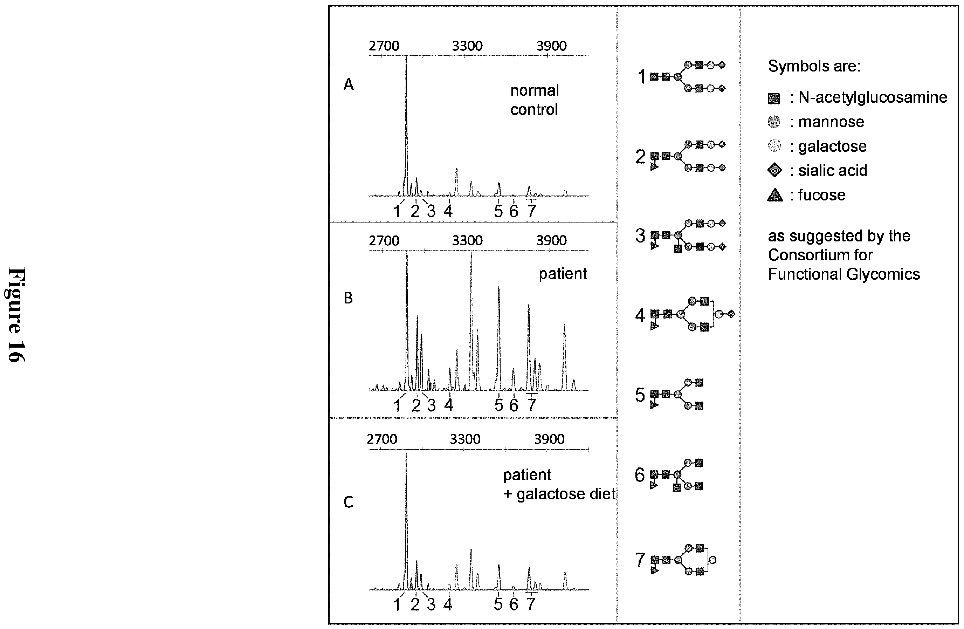

**Figure 16. Dietary Effects of Galactose on total Serum Glycome.**

Panels A-C show the analysis of total serum N-glycome before and during galactose supplementation. The glycans are cut from the proteins and analyzed by capillary electrophoresis on a standard ABI-3130 capillary sequencer. The sample processing has been described completely elsewhere (41), with the only modification that samples were not desialylated. Panel A shows a normal control, panel B a patient before galactose supplementation, C same patient during galactose supplementation. Glycans lacking one or more sialic acid residues only, but missing no galactose, are depicted in red, those lacking also galactose in blue. Peaks 1-3 are fully sialylated glycans. To show the effect of missing galactose all blue peaks were also numbered and detailed structures are given in the right panel. The figure shows that galactose supplementation normalizes to a large extent the glycosylation of total serum glycoproteins (not only transferrin).

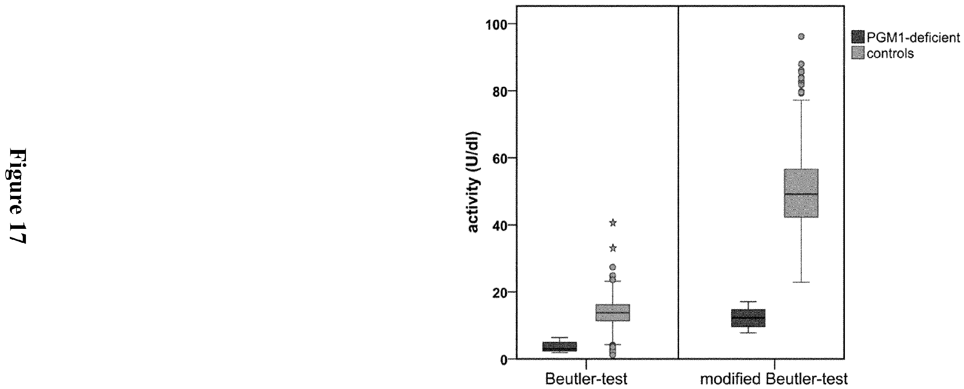

**Figure 17. Performance of Guthrie Screening Assay for PGM1-Deficiency**

Dried blood spots of the GALT kit calibrators and controls, as well as samples from 8 patients with PGM deficiency and anonymised DBS from 2017 healthy newborns were analyzed. Using the unmodified Beutler test (box-plot left panel) as measure of PGM1 activity, intra-and interassay variation was less than 10% for PGM activities between 3 U/dl to 40 U/dl. GALT activity from the 8 patients with PGM deficiency was significantly lower than in the healthy newborns (p = 0.014), however, there was significant overlap (extreme values in controls indicated by asterisks). The addition of PGM from rabbit heart restored the GALT-activity in the DBS of PGM-deficient patients to normal values (Table S4).

When the Beutler test was modified, the difference between PGM activity in DBS from PGM-deficient patients and healthy newborns was highly significant (p < 0.001) with a clear discrimination between the two groups (right panel; Table S4). Using a preliminary cut-off at 0.1%, all PGM1-deficient patients were recognized (sensitivity 100%, specificity > 99.9%). A pilot study will be necessary to determine true and false positive rate in the population in order to determine the positive predictive value of the assay.

In erythrocytes 50 % of PGM-activity is contributed by PGM2. However, this should not interfere with the assay results, since PGM2 has PGM activity only at high concentrations of its cofactor glucose-1,6-bisphosphate (Glc-1,6-BP; in erythrocytes about 590 $\mu$M), while below 190 $\mu$M PGM2 has mainly phosphopentose mutase activity. (19) In our assay volume of 100 $\mu$l there is about 1.5 $\mu$l volume equivalent to erythrocytes. Thus, Glc-1,6-BP is diluted to about 10 $\mu$M. A small contribution of up to 7 $\mu$M may also come from contamination of the substrate glucose-1-phosphate by glucose-1,6-diphosphate (Sigma No. G6875, 0.01-0.2%).

Handling of Guthrie spots is the same as in galactosemia screening. If necessary samples can be stored at -18°C for months without any detectable change in GALT or PGM1-activity.

**EXAMPLESI**

The Examples exemplify the invention:

PATIENTS

We initially identified a pair of male siblings (Patients 1.1 and 1.2) from a consanguineous family (the index family; parents were first cousins) with clinical features suggestive of congenital disorders of glycosylation, including myopathy, hepatopathy, short stature, and hypoglycemia. Analysis of serum transferrin by means of isoelectric focusing was performed to screen for congenital disorders of glycosylation. (3,4) This evaluation revealed an atypical pattern (Fig. 1), suggesting a novel disorder.

Several additional affected persons were identified subsequently. A total of 19 patients from 16 families (including three sibling pairs) were included in the present study, showing clinical features suggestive of congenital disorders of glycosylation and an atypical pattern on isoelectric focusing (Fig. 5 and Table S1 in the Examples II, available with the full text of this article at NEJM.org; some features of Patients 2, 4, 5.1, 5.2, 6, 8, and 9 have been described previously (2,5-8)). Approval by a human subjects committee was obtained for all the clinical studies. Written informed consent was obtained from all the participants or, in the case of children, from a parent or legal guardian.

GENETIC STUDIES

Because the parents of the index family were first cousins and were unaffected, we assumed autosomal recessive inheritance of the disease. We therefore performed homozygosity mapping with DNA from the two affected siblings, using Human1M-Duo BeadChips (Illumina; see the Examples II). Within the regions of homozygosity thus identified, we used whole-exome sequencing to identify sequence variants that might be associated with the disease. After identification of mutations in the gene for phosphoglucomutase 1 (PGM1) in both siblings (see the Examples II), PGM1 in additional patients was analyzed by means of Sanger sequencing (see the Examples II).

TISSUE SPECIMENS

Blood samples and skin-biopsy and muscle-biopsy specimens were obtained from study participants after informed consent had been provided. Leukocytes were obtained from blood samples. Fibroblast cultures were prepared from skin-biopsy specimens from 15 patients (see the Examples II). Biopsy specimens of the vastus lateralis muscle were obtained from 4 patients. Fibroblasts pelleted from cultures, leukocytes, and muscle-biopsy specimens were frozen in liquid nitrogen for use in study assays.

PHOSPHOGLUCOMUTASE 1 EXPRESSION AND ACTIVITY

Total RNA was extracted from fibroblast pellets, and PGM1 messenger RNA (mRNA) was quantified by means of a real-time polymerase chain reaction assay (see the Examples II). Western blot analysis was performed on cytosolic proteins extracted from fibroblast pellets with the use of a monoclonal anti-PGM1 antibody (see the Examples II). Phosphoglucomutase 1 enzyme activity was assayed spectrophotometrically on extracts from fibro- blasts, leukocytes, or skeletal-muscle cells (see the Examples II).

GLYCOSYLATION ASSAYS

Analysis of transferrin glycosylation was performed on serum samples with the use of isoelectric focusing, (3,4) sodium dodecyl sulfate-polyacrylamide-gel electrophoresis (SDS-PAGE), (3) or liquid chromatography-mass spectrometry (9) (Fig. 3, and the Examples II). The cell-surface glycoprotein intercellular adhesion molecule 1 (ICAM-1), the expression of which is markedly reduced by deficient glycosylation, (10) was assayed in fibroblast cultures from patients by means of Western blotting and immunofluorescence analysis with the use of a monoclonal anti-ICAM-1 antibody (see the Examples II). Green fluorescent protein engineered for retention in the endoplasmic reticulum was modified to contain an N-glycosylation site that, when glycosylated, causes loss of fluorescence (Glyc-ER-GFP). (11) Glyc-ER-GFP was transfected into patient fibroblasts in culture, and fluorescence was measured by means of quantitative microscopy (see the Examples II).

SUGAR METABOLITE AND GLYCOGEN QUANTIFICATION

The nucleotide sugars uridine diphosphate (UDP)-glucose and UDP-galactose were extracted from cultured fibroblasts and erythrocytes from patients and were quantified by means of reverse-phase high-performance liquid chromatography (see the Examples II). (12,13) Glucose-1-phosphate was analyzed by means of a photometric method (see the Examples II), and galactose-1-phosphate was assayed with the use of 14C-labeled UPD-glucose. (14) Glycogen was extracted from fibroblasts and digested with amyloglucosidase as described previously. (15) The total amount of glucose was analyzed by means of gas chromatography-mass spectrometry. (16) Glycogen content in fibroblasts from patients was also assessed by means of electron microscopy (see the Examples II).

GALACTOSE SUPPLEMENTATION IN CULTURE

Galactose (Sigma-Aldrich) was added to fibroblast culture medium to increase the concentration of intracellular UDP-galactose by means of the galactose-1-phosphate uridyltransferase (GALT) reaction (Fig. 2). The concentration used was 0.5 mM.

DIETARY SUPPLEMENTATION

Galactose powder was supplied by Falcento. Galactose levels in whole blood were determined in a healthy volunteer after the oral consumption of 250 ml of water in which 0.3 g of galactose per kilogram of body weight had been dissolved. Measurements of galactose levels in blood were made by means of spectrophotometry at intervals of 10 minutes during the first hour and at intervals of 30 minutes for an additional 3 hours. Lactose or galactose supplementation was administered as an aqueous solution at a dose of 0.5 to 1.0 g per kilogram per day, divided into three to six daily doses (on the basis of patient preference).

SCREENING ASSAY

To develop a potential presymptomatic screening test for phosphoglucomutase 1 deficiency, we developed a modified version of the Beutler test, which is used with Guthrie heel-prick test cards (dried blood spots) to screen for galactosemia. The Beutler test is dependent on the generation of glucose-1-phosphate and its metabolites by means of the GALT reaction (Fig. 6 in the Examples II). We substituted glucose-1-phosphate for the substrate in the assay. We also developed a similar assay using glucose- 6-phosphate, which we anticipated would bypass the enzyme defect in phosphoglucomutase 1 deficiency.

RESULTS

CLINICAL PHENOTYPE

A total of 19 affected family members, 3 to 43 years of age, were identified in 16 families (Table S1 in the Examples II). At birth, a bifid uvula with or without cleft palate was the only clinical manifestation.

Subsequent clinical manifestations varied among the patients (Fig. 4). Signs of hepatopathy with moderately elevated serum aminotransferase levels developed in all patients. Dilated cardiomyopathy, cardiac arrest, or both occurred in six patients; three patients were listed for heart transplantation. The majority of the patients had muscle symptoms, including exercise intolerance, muscle weakness, and rhabdomyolysis. Malignant hyperthermia with severe rhabdomyolysis occurred in two patients after the administration of general anesthesia. Growth retardation was reported in all but four patients. Two girls had hypogonadotropic hypogonadism with delayed puberty. Hypoglycemia was common, especially in childhood, requiring treatment with frequent meals, complex carbohydrates, or overnight tube feeding.

TRANSFERRIN GLYCOSYLATION

Transferrin analysis by means of isoelectric focusing, supplemented by SDS-PAGE data, revealed that the affected family members in the other families had a distinctive pattern of glycosylation, as did the two siblings in the index family (Fig. 5 in the Examples II). Mass spectrometry revealed the presence of a variety of transferrin glycoforms, including forms lacking one or both glycans as well as forms with truncated glycans (Fig. 3A). There was considerable variation in the transferrin-glycoform profile among the patients (Fig. 8 in the Examples II).

GENETIC IDENTIFICATION OF PHOSPHOGLUCOMUTASE 1 DEFICIENCY

Homozygosity mapping of the two affected sib- lings in the index family identified autosomal regions of homozygosity totaling 87.5 mega-bases (Fig. 9 in the Examples II). Whole-exome sequencing identified 1516 sequence variants within the regions of homozygosity. These variants were filtered to identify 10 genes carrying homozygous nonsynonymous mutations that had not already been identified as polymorphisms. Two of these genes were considered to be of potential functional relevance, but only the mutation in PGM1 cosegregated with disease. Independently, functional filtering of whole-exome sequencing data for potential CDG-I candidate genes in another patient (Patient 6 in Table S1 in the Examples II) led to the identification of a homozygous PGM1 mutation.

A total of 21 different PGM1 mutations were identified in 16 families. Nine patients were compound heterozygotes; all others were homozygotes. The locations of the individual mutations with respect to the structural domains of the phosphoglucomutase 1 protein are shown in Fig. 10 in the Examples II.

PHOSPHOGLUCOMUTASE 1 EXPRESSION AND ACTIVITY

Quantification of PGM1 mRNA was performed for 11 patients and showed considerable variation in expression (Fig. 11 in the Examples II). Only two homozygous premature stop variants with predicted nonsense-mediated decay showed mRNA levels that were less than 10% of those in healthy controls. Likewise, Western blotting of phosphoglucomutase 1, performed for 13 patients, showed various amounts of protein (Fig. 12 in the Examples II). Assays for phosphoglucomutase 1 enzyme activity, however, showed markedly decreased activity in all 17 patients who were tested, amounting to at most 12% of the activity seen in controls (Table S1 in the Examples II).

SUGAR-METABOLITE AND GLYCOGEN QUANTIFICATION

Analyses of sugar metabolites in fibroblasts from patients revealed increased concentrations of galactose-1-phosphate and glucose-1 phosphate, as compared with fibroblasts from controls. The ratio of UDP-galactose to UDP-glucose was decreased, suggesting that UDP-galactose is limiting for N-glycan biosynthesis (see the Examples II). Glycogen content, as assessed by means of either electron microscopy or gas chromatography-mass spectrometry after amyloglucosidase digestion, was similar to that seen in fibroblasts from controls (see the Examples II).

EFFECT OF GALACTOSE SUPPLEMENTATION IN CULTURE

With the addition of galactose to the culture medium of fibroblasts from patients, glycosylation was substantially enhanced, as assessed by means of glycosylation of ICAM-1 (Fig. 13 in the Examples II) and Glyc-ER-GFP (Fig. 14 in the Examples II). The effect on Glyc-ER-GFP was further enhanced by the addition of uridine to the culture medium, which resulted in essentially normal glycosylation of this test protein (Fig. 14 in the Examples II). Glycogen content was not affected by either galactose or glucose supplementation (Table S3 in the Examples II).

DIETARY SUPPLEMENTATION

Oral consumption of galactose by a healthy volunteer confirmed the bioavailability of this sugar. The plasma concentration of galactose reached a maximum of 0.7 mM at 50 minutes and decreased to baseline levels after 2 hours (data not shown).

Six study participants (Patients 1.1, 1.2, 2, 5.2, 6, and 11 in Table S1 in the Examples II) received dietary supplementation with lactose or galactose. Within 2 weeks, transferrin glycosylation had improved substantially as shown by mass spectrometry (Fig. 3B, and Fig. 15 in the Examples II). In addition, there was substantial improvement in glycosylation of the total serum N-glycome (the set of all N-glycosylated proteins) with galactose supplementation (Fig. 16 in the Examples II).

In Patients 2 and 6, no further episodes of rhabdomyolysis occurred after the initiation of galactose treatment, and cardiac function remained stable. In these same girls, who were 15 and 17 years of age, respectively, hypogonadotropic hypogonadism resolved within a few weeks after the initiation of galactose supplementation. Levels of luteinizing hormone, which were initially at the 2.5th percentile, increased in these two patients by a factor of 5 and a factor of 10, respectively; in addition, levels of follicle- stimulating hormone (FSH) normalized, and clinical signs of puberty appeared. Ultrasonography of the liver, which was performed every 3 months in Patients 2 and 6 during galactose supplementation, showed no increase in echogenicity and no signs of cholestasis.

SELECTIVE SCREENING

We used the standard Beutler test for galactosemia as well as two modified versions to determine whether such a test could reliably identify patients with phosphoglucomutase 1 deficiency (Fig. 6 in the Examples II). In the standard Beutler test, the activity of the GALT pathway was significantly lower in eight study patients with phosphoglucomutase 1 deficiency than in healthy newborn controls (Fig. 17 and Table S4 in the Examples II). The addition of phosphoglucomutase 1 from rabbit heart restored the activity of the GALT pathway in the patients to normal levels.

In a modified Beutler test with the use of glucose-6-phosphate, which is dependent on the activity of glucose-6-phosphate dehydrogenase, there was no significant difference between study patients and controls. However, in a modified Beutler test with the use of glucose-1-phosphate, which is dependent on the activity of phosphoglucomutase 1, there was a significant reduction in enzyme activity, as assessed by means of NADPH fluorescence, in the patients (Fig. 17 and Table S4 in the Examples II).

In our study, we found that phosphoglucomutase 1 deficiency, which has been shown to be a glycogen storage disorder, is also a mixed-type congenital disorder of glycosylation. We identified 21 different mutations in PGM1 in 19 patients. Although all the patients had multisystem disease at the time of the study, the only apparent clinical feature at birth was a bifid uvula (in 16 of the 19 patients).

Extensive use of common protein polymorphisms of phosphoglucomutase 1 in paternity testing led to the discovery of a case of reduced enzyme activity in 1963, and since then additional cases of reduced phosphoglucomutase 1 activity have been described sporadically. (8,17-22) In 2009, data on an adult with exercise-induced muscle cramps and episodes of rhabdomyolysis (Patient 8 in the current study) were reported. (2) Abnormal glycogen accumulation was noted in the muscles, the level of phosphoglucomutase 1 activity was only 1% of the normal level, and mutations in PGM1 were identified. The disease was designated as glycogenosis type XIV. However, the broad clinical and biochemical spectrum of phosphoglucomutase 1 deficiency remained unrecognized.

In contrast to many other congenital disorders of glycosylation, in which patients have psychomotor retardation, the brains of our patients were not affected. This is probably explained by the fact that other isoenzymes substitute for phosphoglucomutase 1 in the brain (see the Examples II).

We did not attempt to show experimentally the mechanisms that account for the clinical phenotypes of phosphoglucomutase 1 deficiency. However, analysis of the literature provides at least three different mechanisms. Hypoglycemia, lactic acidosis, and exercise intolerance are presumably consequences of disordered glucose metabolism. After a dietary glucose load, large amounts of glucose-6-phosphate are generated, which cannot be converted into glycogen. Thus, a substantial portion is diverted to lactate and fatty acid production (Fig. 2). During fasting, liver glycogen cannot be converted into glucose via glucose-6-phosphate, and hypoglycemia may occur. The inability of skeletal muscles to quickly use glycogen for the anaerobic production of energy leads to exercise intolerance and potentially to rhabdomyolysis. These characteristics are also seen with glycogen storage disease la, in which there is a defect in hepatic glucose-6-phosphatase. Stabilizing glucose homeostasis by means of dietary intake of complex carbohydrates (23) and restricting exercise to a level below the aerobic threshold might be beneficial.

Two endocrinologic features of phosphoglucomutase 1 deficiency may be directly linked to defective glycosylation. First, Patients 2 and 6 had hypogonadotropic hypogonadism without clinical manifestations of puberty. The gonadotropins and their receptors are glycoproteins, and their function has been shown to be reduced by inadequate glycosylation. (24) Second, 15 patients had short stature, although the growth hormone (somatotropin) level was normal to high in the patients in whom it was measured. However, levels of insulin-like growth factor 1 (IGF-1) and IGF-binding protein 3 were low, as they are in patients with CDG-Ib, a glycosylation disorder in which dietary supplementation with mannose improves glycosylation and growth. (3,25) A detailed discussion of the literature concerning the role of glycosylation in the gonadotropin and IGF-1 systems is provided in the Examples II.

A third mechanism relates PGM1 mutations to dilated cardiomyopathy. Phosphoglucomutase 1 binds to the heart-muscle-cell-specific splice variant of ZASP (Z-band alternatively spliced PDZ-motif protein), and ZASP mutations that affect the binding of phosphoglucomutase 1 are associated with dilated cardiomyopathy. (26,27) Our study shows that defects in PGM1 are also associated with dilated cardiomyopathy. A detailed discussion of this mechanism is provided in the Examples II.

We also found that galactose supplementation can improve glycosylation both in fibroblast-cell culture and in patients. The extent to which this improvement in glycosylation mitigates the clinical features of phosphoglucomutase 1 deficiency was not investigated systematically in our study. In the six patients in this study who received dietary supplementation with galactose, some potentially important clinical changes were seen. In the two girls with hypogonadotropic hypogonadism, the levels of luteinizing hormone increased markedly, FSH levels normalized, and clinical signs of puberty appeared. However, a systematic clinical study of the effects of galactose supplementation will be necessary to determine the extent to which such therapy can correct the clinical features of this condition; such studies will also incidentally help to define the pathogenesis of the individual elements of the clinical syndrome of phosphoglucomutase 1 deficiency.

We devised a clinical test for the diagnosis of phosphoglucomutase 1 deficiency, which was based on the Beutler test for galactosemia. On initial evaluation, this modified Beutler test appeared to discriminate effectively between persons with phosphoglucomutase 1 deficiency and controls. However, validation is required before the assay can be recommended for routine clinical use.

In conclusion, we found that phosphoglucomutase 1 deficiency, previously identified as a glycogen storage disorder, is also a mixed-type congenital disorder of protein N-glycosylation. The presence of a bifid uvula at birth may be an early clinical clue to the presence of this syndrome. Supplementation with galactose leads to biochemical improvement in indexes of glycosylation. The degree to which galactose supplementation may lead to clinical improvement in the disease syndrome is not yet established.

REFERENCES

1. Quick CB, Fisher RA, Harris H. A kinetic study of the isozymes determined by the three human phosphoglucomutase loci PGM1, PGM2, and PGM3. Eur J Bio- chem 1974;42:511-7.

2. Stojkovic T, Vissing J, Petit F, et al. Muscle glycogenosis due to phosphoglucomutase 1 deficiency. N Engl J Med 2009;361:425-7.

3. Niehues R, Hasilik M, Alton G, et al. Carbohydrate-deficient glycoprotein syndrome type Ib: phosphomannose isomerase deficiency and mannose therapy. J Clin Invest 1998;101:1414-20.

4. de Jong G, van Noort WL, van Eijk HG. Optimized separation and quantitation of serum and cerebrospinal fluid transferrin subfractions defined by differences in iron saturation or glycan composition. Adv Exp Med Biol 1994;356: 51-9.

5. Mandato C, Brive L, Miura Y, et al. Cryptogenic liver disease in four children: a novel congenital disorder of glycosylation. Pediatr Res 2006;59:293-8.

6. Mohamed M, Guillard M, Wortmann SB, et al. Clinical and diagnostic approach in unsolved CDG patients with a type 2 transferrin pattern. Biochim Biophys Acta 2011; 1812:691-8.

7. Gehrmann J, Sohlbach K, Linnebank M, et al. Cardiomyopathy in congenital disorders of glycosylation. Cardiol Young 2003;13:345-51.

8. Timal S, Hoischen A, Lehle L, et al. Gene identification in the congenital disorders of glycosylation type I by whole-exome sequencing. Hum Mol Genet 2012;21:4151-61.

9. Biffi S, Tamaro G, Bortot B, Zamber- Ian S, Severini GM, Carrozzi M. Carbohydrate-deficient transferrin (CDT) as a biochemical tool for the screening of congenital disorders of glycosylation (CDGs). Clin Biochem 2007;40:1431-4.

10. He P, Ng BG, Losfeld ME, Zhu W, Freeze HH. Identification of intercellular cell adhesion molecule 1 (ICAM-1) as a hypoglycosylation marker in congenital disorders of glycosylation cells. J Biol Chem 2012;287:18210-7.

11. Losfeld ME, Soncin F, Ng BG, Singec I, Freeze HH. A sensitive green fluorescent protein biomarker of N-glycosylation site occupancy. FASEB J 2012;26:4210-7.

12. Räbinä J, Mäki M, Savilahti EM, Järvinen N, Penttilä L, Renkonen R. Analysis of nucleotide sugars from cell lysates by ion-pair solid-phase extraction and reversed-phase high-performance liquid chromatography. Glycoconj J 2001;18:799- 805.

13. Nakajima K, Kitazume S, Angata T, et al. Simultaneous determination of nucleotide sugars with ion-pair reversed-phase HPLC. Glycobiology 2010;20:865-71.

14. Shin YS. Galactose metabolites and disorders of galactose metabolism. In: Hommes FA, ed. Techniques in diagnostic human biochemical genetics. New York: Wiley-Liss, 1991:267-83.

15. Passonneau JV, Lauderdale VR. A comparison of three methods of glycogen measurement in tissues. Anal Biochem 1974;60:405-12.

16. Sickmann HM, Schousboe A, Fosgerau K, Waagepetersen HS. Compartmentation of lactate originating from glycogen and glucose in cultured astrocytes. Neurochem Res 2005;30:1295-304.

17. Perez B, Medrano C, Ecay MJ, et al. A novel congenital disorder of glycosylation type without central nervous system involvement caused by mutations in the phosphoglucomutase 1 gene. J Inherit Metab Dis 2013;36:535-42.

18. Thomson WH, MacLaurin JC, Prineas JW. Skeletal muscle glycogenosis: an investigation of two dissimilar cases. J Neurol Neurosurg Psychiatry 1963;26:60-8.

19. Sugie H, Kobayashi J, Sugie Y, et al. Infantile muscle glycogen storage disease: phosphoglucomutase deficiency with decreased muscle and serum carnitine levels. Neurology 1988;38:602-5.

20. Illingworth B, Brown DH. Glycogen storage diseases, types III, IV, and VI. In: Whelan WJ, Cameron MP, eds. Control of glycogen metabolism (Ciba Foundation Symposium). London: J. & A. Churchill, 1964:336-53.

21. Illingworth Brown B, Brown DH. Phosphoglucomutase deficiency as a possible cause of glycogenosis. In: Dickens F, Randle PJ, Whelan WJ, eds. Carbohydrate metabolism and its disorders. New York: Academic Press, 1968:144-6.

22. Nakashima H, Suo H, Ochiai J, Sugie H, Kawamura Y. A case of adult onset phosphoglucomutase deficiency. Rinsho Shinkeigaku 1992;32:42-7. (In Japanese.)

23. Weinstein DA, Wolfsdorf JI. Effect of continuous glucose therapy with uncooked cornstarch on the long-term clinical course of type 1a glycogen storage disease. Eur J Pediatr 2002;161:Suppl 1:S35-S39.

24. Huhtaniemi IT. Polymorphism of gonadotropin action: clinical implications. Asian J Androl 2000;2:241-6.

25. Miller BS, Khosravi MJ, Patterson MC, Conover CA. IGF system in children with congenital disorders of glycosylation. Clin Endocrinol (Oxf) 2009;70:892-7.

26. Arimura T, Inagaki N, Hayashi T, et al. Impaired binding of ZASP/Cypher with phosphoglucomutase 1 is associated with dilated cardiomyopathy. Cardiovasc Res 2009;83:80-8.

27. Vatta M, Mohapatra B, Jimenez S, et al. Mutations in Cypher/ZASP in patients with dilated cardiomyopathy and left ventricular non-compaction. J Am Coll Car- diol 2003;42:2014-27.

**EXAMPLES II Homozygosity mapping: mapping the disease locus by linkage analysis**

For homozygosity mapping, 400 ng DNA from each of the two affected sibs of the inbred pedigree shown in Fig. 9A was used. Illumina 1M Duo beadchips were processed according to the manufacturer's instructions. The data presentation by Illumina Genomestudio software enabled us to depict the borders (RS-IDs of markers) of homozygous regions by visual inspection. In addition we calculated lod scores over the chromosomes using Merlin-1.1.2 software.[1] The result for chromosome 1 is shown in Fig. 9B and the other chromosomes follow in Fig. 9C.

Since in the pedigree the effective number of meioses is 6, a maximum lod score value below $\log_{10}(2^6)$ = 1.8062 could be expected (limit for disease freq $\to$ 0 and SNP allele frequencies $\to$ 0). After providing Merlin with quality filtered Illumina-data from the two affected sibs (-380000 SNPs), the pedigree structure, real SNP-allele frequencies, and a model file for recessive disease with disease frequency set to 0.0001, lod scores were calculated. A maximum lod score of 1.8057 was obtained. Lod score results below -12 were set to -12. Data were further processed using visual basic code to extract all those homozygous regions with a size of more than 1 Mb. To this end, the lod scores were dichotomized (1 vs. 0) using a cutoff 1 lod unit below the maximal limit of 1.8062, i.e. 0.8. Then the homozygous regions were determined as regions of at least 1 Mb containing no "0" and finally the first heterozygous marker adjacent to each end of a homozygosity region was selected as the limit of the homozygous region. Occurrence of two singular heterozygous SNPs, one in each of two large homozygous regions, (15 Mb on chr. 1, and 40.6 Mb on chr. 10) were not considered as interruption of the homozygous regions. This conclusion is based on the normal human mutation frequency (1-2.5/$10^8$ basepairs per meiosis[2,3]) and the pedigree structure (distance of 4 meioses between the parents of the affected sib pair); with these parameters about 0.6-1.5 mutations/15 Mbases present in one of the parents are expected. One of these SNPs is visible in Fig. 9B about three megabases downstream of the PGM1 locus as a spike, down to a lod score < -8. By the homozygosity mapping, the candidate region could be reduced to 87.37 MB using actual Ensembl-release 69 data, scattered over the autosomal chromosomes in 12 regions.

**Whole exome sequencing: identification of defective *PGM1***

For whole exome sequencing, genomic DNA was isolated from 8 ml of blood using PAXgene Blood DNA system (www.preAnalytiX.com). DNA quality was checked photometrically, and concentration was measured using the Qubit system (Qubit 2.0 fluorometer). DNA was processed following the Agilent SureSelect protocol. 3 $\mu$g DNA were sheared using a Covaris S2 (Covaris, USA) to obtain fragments around 200 bp. The Agilent protocol for selection of the exonic sequences was performed almost exactly as given, except that a BioRad Experion was used for QC steps during the process, including calculation of the final concentration of the library. A 10 nM sample-DNA dilution in Tris-HCl 10 mM, pH 8.5 with 0.1% Tween 20 buffer was further processed according to Illumina protocols, spiked with 1% PhiX-DNA as internal quality control. Cluster generation was performed on the flowcell using an Illumina cBot. Next generation sequencing was started on an Illumina HiScanSQ (paired end mode, 2 x 101 bases). Primary output (.bcl files) was transformed to -qseq.txt files using the Illumina BCL-converter. These files were mapped to the reference sequence comprising PhiX and the whole human genome (hg19), but excluding PAR regions on the Y-chromosome, using CLC Bio software. After SNP-calling, the respective data were exported and reimported into a Microsoft access database.

62973 variants from CLC were obtained. Using a table containing the limits of the candidate regions obtained by homozygosity mapping, the 1516 variants in these regions were selected and exported into a text file suitable for analysis by the Seattle SNP annotation software (http://gvs.gs.washington.edu/SeattleSeqAnnotation/). 1506 of the variants were single base substitutions comprising 164 missense, 2 nonsense, and 0 splice site mutations. 10 variants were indels (insertion/deletion mutations). Regarding the indels, one was false positive due to read-through into the primer region, three were known variants, one fitting 100% to alternate assembly and two with reported minor allele frequencies of 18 and 49 % respectively. The 1506 single nucleotide substitutions were further analyzed for minor allele frequency (MAF), and all showing MAF > 1.4% were removed (corresponding homozygosity freq. $0.014^2 \approx 1/5000$ i.e. definition of rare diseases). To determine the minor allele frequencies, HapMap-frequencies and Exome Sequence Project-data (automatically extracted via http://snp.gs.washington.edu/SeattleSeqAnnotation134/) as well as MAF-data obtained via Biomart from the Ensembl database were used. 168 variant positions remained after application of the MAF > 1.4% cutoff. Next, we filtered off variants that were designed as intron / intergenic / outside coding / coding-synonymous etc., and those where Chimp allele was same as patients sample allele were also excluded. By this procedure the dataset was reduced to 10 variants in 10 genes (HGNC-symbols): *PGM1, LRRC40, PAPOLG, RIOK1, GCNT6, PDE6C, HPSE2, DCLRE1A, PDZD8, PRDX3*.

The functional description suggested a relation to glycosylation for *PGM1* and GCNT6 only. Inheritance of both variants was checked in the family. The *PGM1* variant was homozygous in the two affected patients only (parents were heterozygotes, Fig. 9A), while the *GCNT6*-variant was also detected in homozygous form in an unaffected sib. We confirmed *PGM1* as the defective gene in additional individuals with similar IEF-patterns. All identified mutations were not reported in dbSNP or Exome Sequencing Project of 6500 exomes. Functional relevance was demonstrated by a strong reduction in PGM1 enzymatic activity for all patients in respective material (fibroblasts, muscle cells, lymphocytes; see activity results in Table S1).

In an independent approach, genomic DNA of patient 6 was extracted from fibroblast pellets according to the manufacturer's protocol using a Qiagen Mini kit (Qiagen), and was checked for DNA degradation on agarose gels. The exome was captured and enriched using a SureSelect human exome enrichment kit (Agilent). After amplification, samples were sequenced on a Solid4 sequencer (Applied Biosystems) and color space reads were iteratively mapped to the hg19 reference genome with the Solid Bioscope software version 1.3, as described previously.[4,5] After filtering against known SNPs and prioritization using a broader list of genes that included known CDG genes and additional other genes involved in sugar and nucleotide sugar metabolism[4], *PGM1* stood out as a primary candidate gene and was confirmed by enzyme activity measurement.

**PCR and sequencing for mutation analysis**

For sequencing of additional CDG patients, primers for genomic DNA and cDNA were designed with Primer3 software.[6] The samples, containing 1$\mu$l DNA (concentration range 10-80 ng/$\mu$l), 2 $\mu$l PCR Buffer 10x (Qiagen), 2 mM dNTPs (GE Healthcare, Buckinghamshire, UK), 9 $\mu$l A. dest., 0.1 $\mu$l Taq DNA Polymerase (Qiagen), 1 $\mu$l primer forward and 1 $\mu$l primer reverse (20 pmol/$\mu$l), were cycled 35 times using the primers and conditions listed in Table S2.1 and S2.2 (Examples II). Purification of the amplified DNA was done according to the manufacturer's protocol of PCR Product Pre-Sequencing Kit (USB Corporation, Ohio, USA).

Sanger sequencing was performed using Big Dye Terminator Kit 3.1 (Applied Biosystems, Darmstadt, Germany) and MicroAmp Optical 96-Well Reaction Plate (Applied Biosystems) on an ABI 3730 XL capillary sequencer after purification with Sephadex on MultiScreen Filter Plates (Millipore Corporation, Billerica, USA).

**Patient cells and tissues**

**Leukocytes** were prepared from blood obtained by venipuncture for standard clinical laboratory measurements. For phosphoglucomutase 1 activity, **leukocyte** pellets were isolated from heparin blood by dextran precipitation according to routine laboratory protocols. Pellets were washed with PBS and frozen at -20 °C.

**Fibroblasts** were obtained by standard procedures from skin biopsies. In brief, a 2 - 4 mm skin punch are obtained under local anesthesia (EMLA-plaster, AstraZeneca) and added to a 15 ml centrifuge tube with 10 ml cell culture medium (MEM containing 1 g glucose/L, 1% of L-glutamine/streptomycin/penicillin mixture, and 10 % FBS; PAA laboratories). Under the sterile conditions, medium is removed and the specimen flushed with a small volume of fresh medium into a 10 cm petri dish. The specimen is cut into 4-6 small pieces, that are placed into a 25 cm$^2$ cell culture flask with the subcutaneous fat region touching to the plastic surface. The flask is turned into a vertical position, filled with 5 ml culture medium and left in the vertical position for about 2 h in the incubator (37°C, 5% CO$_2$) to allow the skin pieces to get firm contact to the flask surface. Afterwards the flask is gently turned into horizontal position. Fibroblasts are allowed to grow out from tissue for 3-4 weeks, changing the medium once per week. The cells grown out from the tissue pieces are trypsinized, distributed within the same flask and allowed to further grow to confluence. From that point cells are split 1:3 about every 4 days. Cells are cryopreserved in 10 % DMSO / culture medium and placed at liquid nitrogen for long term storage.

To prepare cell pellets for later use in measurement of phosphoglucomutase 1 activity or nucleotide sugar analysis, cells were grown in culture to 80-90% confluence, were then trypsinized, pelleted, washed with PBS and pelleted again at 500 x g, 5 min. After removal of supernatant, pellets were stored at -20°C or in liquid nitrogen.

Further processing of cells for specific methods is described in the respective paragraphs for each of those methods.

Muscle biopsies were taken from vastus lateralis in a diagnostic setting, before PGM1 deficiency was identified. For later processing and measurements of enzyme activities muscle specimens were washed once with PBS and frozen in liquid nitrogen.

Liver biopsies were obtained for clinical reasons by standard procedures and were taken before the diagnosis of PGM1 deficiency was made.

**Real-time PCR for quantification of PGM1 mRNA**

An equivalent of 25 ng total cellular RNA was mixed with 9 $\mu$l Qiagen-Quantitect 2xSybr-Green real time PCR kit in a total volume of 18 $\mu$l; each primer was added at 0.2 $\mu$M final concentration. Triplicate measurements were performed in an ABI3700 realtime cycler (Applied Biosystems/Invitrogen) programmed at15 min 95°C, 5 x (15 sec 95°C, 30 sec 60°C, 30 sec 72°C), 40 x (15 sec 95°C, 1 min 60°C, 30 sec 72°C). Primers used for normalization were for *PPIA* from Biomol. *PGM1* primers spanning Exon8-Exon9 were 5'-AAAGATGGACTGTGGGCTGTCCT (SEQ ID NO: 10) and 5'-CCTCAGCTTCCACCTCCTCGTAA (SEQ ID NO: 11).

**Western blotting for PGM1**

For Western blots, fibroblast pellets were washed in PBS (phosphate buffered saline; PAA, Cölbe, Germany), centrifuged (5 min, 500 x g, room temperature) and resuspended in HEPES-sucrose-buffer (0.2 M sucrose, 0.02 M HEPES, pH 7.3) supplemented with protease inhibitors (Complete, Roche; 10 $\mu$g/ml pepstatin A; 10 $\mu$g/ml PMSF). Fibroblasts were disrupted with a Dounce homogenizer. Homogenate was centrifuged at 3000 rpm at 4 °C for 15 min to make the cytosolic fraction, whose protein concentration was determined using Bradford assay. Sample was adjusted to 0.5 $\mu$g/$\mu$l with loading buffer, heated at 95°C for 5 min and 2 $\mu$g of each sample was used for SDS-PAGE (10 % solving gel). PageRuler protein ladder (Fermentas, St. Leon-Rot, Germany; SM0671) served as a molecular weight standard.

After separation, proteins were transferred to Immobilon-P filters, (Millipore GmbH, Schwalbach/Ts., Germany) via Trans-Blot SD Semi-Dry Transfer Cell (Bio-Rad Laboratories GmbH, Munich, Germany) for 1 h at 10 V.

Membranes were blocked overnight with 5 % milk powder (Carl Roth GmbH, Karlsruhe, Germany) in PBST buffer (PBS supplemented with 0.1 % Tween; Merck Schuchardt OHG, Hohenbrunn, Germany) at 4°C, and incubated for 3 hr with 1:2000 diluted monoclonal anti-PGM1 antibody (ab55616; abcam, Cambridge, UK) in PBST (+ 5 % milk powder). After washing 3 x 10 min with 20 ml PBST each, the membrane was incubated with a 1:2000 dilution HRP conjugated polyclonal rabbit anti-mouse antibody (Dako, Glostrup, Denmark; P0260) in PBST (+ 5 % milk powder) for 45 min at room temperature. 4 x 10 min washes at room temperature was followed by the detection of PGM1 using Immobilon Western Chemiluminescent HRP Substrate (Millipore Corporation, Billerica, USA) on a high performance chemiluminescence film (Amersham, GE Healthcare, Munich, Germany). Exposure time was approximately 10 min.

**Phosphoglucomutase 1 enzyme activity**

Cell extracts were prepared from **fibroblasts** or **leukocytes** by thawing a frozen cell pellet in 200 $\mu$l of 20 mM Hepes, pH 7.4, containing 1 mM dithiothreitol, 5 $\mu$g/ml leupeptin and 5 $\mu$g/ml antipain. The cell homogenates were centrifuged for 2 min at 10,000 x g and supernatants used for enzymatic and protein assays. Protein was assayed with the Lowry method[7] using bovine serum albumin as a standard. Phosphoglucomutase was assayed spectrophotometrically at 30 °C, in assay mixtures comprising 100 mM Tris, pH 7.4, 7.5 mM MgCl$_2$, 25 mM KCl, 1 mM dithiothreitol, 0.25 mM NADP and 0.35 U of yeast glucose-6-phosphate dehydrogenase. The phosphoglucomutase assay also included 1 mM glucose-1-phosphate and 10 $\mu$M glucose-1,6-bisphosphate. Blanks were run in the absence of the substrate. The assay was initiated by the addition of an appropriate volume (2-5 $\mu$l) of an extract containing 5-15 mg protein/ml.

Similarly PGM1-activity from **muscle tissue** was measured with following modifications: Muscle specimens previously stored in liquid nitrogen were thawed. Muscle homogenate (1:10 w/v) was prepared in a buffer composed of 0.025 mM triethanolamine-hydrochloride, 1.25 mM EDTA, 1 mM $\beta$-mercaptoethanol, pH 7.5, with mortar and pestle. After centrifugation (5 min at 8,000 x g), the supernatant was 1:4 diluted in the same buffer.

Phosphoglucomutase was assayed spectrophotometrically at 340 nm and at 25°C. Twenty-five $\mu$l of muscle homogenate were mixed with 1975 $\mu$l of a mixture containing (final concentrations) EDTA (4.2 mM), MgCl$_2$ (10 mM), NADP (0.57 mM), glucose-1,6-diphosphate (15 $\mu$M), glucose-6-phosphate dehydrogenase (875 U/l), glucose-1-phosphate (3.5 mM) and triethanolamine-hydrochloride buffer pH 7.5 (85 mM). Blanks were run in the absence of substrate. The assay was initiated with glucose-1-phosphate.

**Assays of transferrin glycosylation**

The standard assay for measuring transferrin glycosylation is **isoelectric focusing,** which can show a deficiency in protein N-glycosylation, due to a reduced number of the terminal sialic acids. Interpretation of results is supported by **SDS-PAGE** which shows changes in the protein mass of transferrin, if whole glycans are missing, i.e. the method indicates unoccupied glycosylation sites. **QTOF mass spectrometry of intact transferrin** can reveal more detailed changes in the glycan structure.

**Isoelectric focusing of transferrin**

Isoelectric focusing of serum transferrin (IEF) is used as a measure for the general efficiency of the protein N-glycosylation process. Transferrin is a glycoprotein produced in the liver containing two N-linked oligosaccharides with two negatively charged terminal sialic acids each (tetrasialotransferrin). Due to its electric charge, transferrin migrates through an agarose gel (agarose for IEF, GE Healthcare Bio Sciences AB, Uppsala, Sweden) prepared with ampholines with a pH range of 5.0-7.0 (GE Healthcare Bio Sciences AB) and coated on Gel Fix plastic sheets (gel size 6.2 x 5 cm$^2$,

Serva Electrophoresis GmbH, Heidelberg, Germany). To produce the gel, 2.2 ml of 1 % agarose with 110 $\mu$l ampholine, solved by heating is casted on the plastic support. Transferrin has two iron binding sites, which should be saturated with iron, so that different states of iron load cannot influence its migration behavior. Therefore, 10 $\mu$l serum are incubated with 66.6 $\mu$l 0.9% NaCl, 2.3 $\mu$l 10 mM Fe(III)citrate and 2.3 $\mu$l 0.1 M NaHCO$_3$. The iron-saturated samples are applied to the IEF-gel automatically via the PhastSystem sample applicator (about 1 $\mu$l/sample) and separated by isoelectric focusing with the Pharmacia Phast system (Pharmacia Fine Chemicals, Uppsala, Sweden), The program for electrophoresis is 300 V for 40 Vh, 100 V for 10 Vh and 200 V for 65 Vh, cooled to 10 °C. For evaluation of data, healthy controls, CDG-I- and CDG-II samples may be loaded for comparison. The healthy controls have tetrasialotransferrin as the major band. The mechanism and effects of mutations leading to CDG-I and -II are explained in Fig. 1.

For visualizing the bands the gel is carefully over layed with 80 $\mu$l of transferrin antibody (Polyclonal Rabbit Anti-Human Transferrin, A0061 Dako A/S, Gostrup, Denmark; undiluted, 15-30 min incubation) to allow building of transferrin-antibody complex precipitates within the gel. Afterwards other proteins are washed out by at least 1 h shaking or overnight incubation in 0.9 % sodium chloride solution in water. After a further wash for 10-30 minutes in distilled water, the gel is dried by application of blotting papers and weight for 0.5-1 h. Further drying is accomplished by application of a hair dryer for up to 1 min. For staining, the gel is incubated for 10 - 30 minutes in a solution of 1.5 g Coomassie R 250 in 300 ml destain (350 ml ethanol, 100 ml acetic acid, 650 ml distilled water). To remove excessive stain, the gel is destained with destain-solution 3 times for 10 min or more often until background color is removed and the transferrin signals are clearly visible.

## Immunoprecipitation and SDS-PAGE of transferrin

5 $\mu$l serum was immunoprecipitated with 1 ml MNT buffer (20 mM 2-[N-morpholino]ethane sulfonic acid, 100 mM NaCl, 30 mM Tris-HCI, pH 7.5) containing 1 mg/ml albumin and 1 mM Fe(III)citrat, 3 $\mu$l rabbit anti-human transferrin antibody (DAKO, 1:1 with glycerin; A0061; DAKOPATTS, Copenhagen, Denmark) with 70 $\mu$l Protein-A-Sepharose 10 % (Sigma-Aldrich Chemie GmbH, Steinheim, Germany) and protease inhibitors (10 $\mu$g/ml of chymostatin, leupeptin, antipain and pepstatin A each). Samples rotated for 4 hours at 4 °C and were then washed once with MNT buffer and afterwards twice at room temperature with 1 ml 0.05% TX-100, 0.1 % SDS, 0.3 M NaCl, 0.02 % NaN$_3$, 10 mM Tris, pH 8.6.

40 $\mu$l sample buffer (45 g 9 % SDS, 75 g 15 % glycerol, 15 ml 1M 30 mM Tris-HCl pH 7.8, 0.25 g 0.05 % bromphenolblue, 500 ml H$_2$O) was added to the immunoprecipitated samples. After boiling, this was run on a 7.5 % polyacrylamide gel (Rotiphorese Gel30, Carl Roth, Karlsruhe, Germany) at 150V in an electrophoresis chamber (Se250 Mighty Small II; Hoefer Scientific Instruments, San Francisco, USA). Afterwards, the gel was stained in 0.5 g Coomassie Blue R 250 (Sigma-Aldrich, Steinheim, Germany) dissolved in 800 ml methanol, 800 ml H$_2$0 and 200 ml 100 % acetic acid.

## QTOF mass spectrometry of intact transferrin

For high-resolution mass spectrometry of transferrin (Fig. 3), a 5 $\mu$l serum sample was incubated with anti-transferrin beads as described.[8] The eluate was analyzed on a microfluidic 6540 LC-chip-QTOF instrument (Agilent Technologies) using a C8 protein chip. Data analysis was performed using Agilent Mass Hunter Qualitative Analysis Software B.04.00. The Agilent BioConfirm Software was used to deconvolute the charge distribution of raw data to reconstructed mass data. For confirmation of the structural annotations, samples were processed for analysis of tryptic transferrin peptides by MALDI-TOF as described[9] (results not shown).

## Western blotting for ICAM-1

Western blotting of ICAM-I (Fig. 13) was performed as described.[10] In brief, approximately 20 $\mu$g of protein lysate from each sample were separated on SDS-PAGE and then transferred to nitrocellulose membranes. After blocking, the membranes were incubated with Goat Anti-mouse ICAM-1 antibody (AF796, R&D Systems, Minneapolis, MN) at 1:500 dilution. Following intensive washing, the membranes were developed with secondary antibodies and visualized using ECL system with higher sensitivity (Immun-Star WesternC Kit, Bio-Rad, Hercules, CA).

## Assay for cellular glycosylation efficiency using modified GFP

Glycosylation efficiency was measured using Glyc-ER-GFP constructs as described.[11] In brief, cells were seeded in a 96 wells-plate at 40% confluence. 24h later, they were incubated in medium without or with addition of supplements (e.g. 0-1 mM galactose +/-100$\mu$M uridine) for 2 days with medium replacement every day. Cells were then transfected by the Glyc-ER-GFP and maintained in the same culture media for two more days. Cells were fixed using 4% paraformaldehyde/4% sucrose and stained by Hoechst 33342 (Thermo scientific). Plates were analyzed using a high-content microscope, IN Cell 1000 (GE Healthcare), and data were analyzed with the CytoSeer software (Vala Sciences) using a protein mask.

### Nucleotide sugar assay

Nucleotide sugars were extracted from fibroblast pellets that had been stored at -20 °C. 900 $\mu$l ice cold 85 % ethanol and 120 $\mu$l PBS were added, and after sonication, cellular debris was removed by centrifugation at 13000 rpm, 10 min. The supernatant was evaporated in a SpeedVac concentrator and resuspended in 1 ml water. Insoluble material was removed by centrifugation and supernatant mixed with 50 $\mu$l 0.2 M $NH_4HCO_3$. The sample was further purified by an Envi-Carb carbon column according to the method of Rabina[12] and UDP-galactose and UDP-glucose were measured by reverse phase HPLC on an Inertsil ODS-4 column according to Nakajima at 254 nm.[13]

### Phosphorylated sugar assay

**Galactose-1-phosphate** was exactly measured as described by Shin YS.[14].

For measurement of **glucose-1-phosphate,** fibroblast cell extract was prepared according to Donthi et al.[15]. Glucose-1-phosphate was measured by employing a coupled enzyme assay using phosphoglucomutase 1 and glucose-6-phosphate dehydrogenase with a fluorescent readout. Briefly, 20 $\mu$l cell extract for each sample was dispensed in the wells of a black assay plate. Depending on the number of samples, two sets of reaction mix were prepared containing 50 mM HEPES, 1 mM magnesium chloride, 100 mM NADP, 10 mM resazurin, 0.25 units diaphorase, 10 mM glucose-1,6-bis-phosphate in a total volume of 80 $\mu$l per sample. Phosphoglucomutase 1 and glucose-6-phosphate dehydrogenase (0.25 units each/sample) were added to one set (F2) and only glucose-6-phosphate dehydrogenase (0.25 units each/sample) was added to the other set (F1). Another mix (Fb) containing glucose-6-phosphate dehydrogenase, but devoid of NADP was prepared to determine the background due to endogenous NADP in each sample. 80 $\mu$l of the reaction mix was added to the respective wells containing 20 $\mu$l of the extract and incubated in dark at room temperature. Fluorescence was recorded at excitation wavelength 530 nm and emission at 590 nm using Flexstation III (Molecular Devices, Sunnyvale, CA, USA). Background fluorescence (Fb) was subtracted from all the samples (F2 and F1). Difference between the fluorescence values (F2-F1) was used to calculate pmols of glucose-1-phosphate from the standard curve generated by known amounts. The results of reaction set F1 were used to calculate **glucose-6-phosphate** levels.

### Biochemical quantification of glycogen

Glycogen was extracted from fibroblasts and digested with amyloglucosidase according to the methods of Passonneau.[16] Total amount of glucose was analyzed by GC/MS. Glucose derived from glycogen was calculated as difference compared to non-treated samples. Results are expressed as glycosyl unit per mg protein according to the method by H.M. Sickmann.[17]

### Evaluation of glycogen by electron microscopy

For electron microscopy, skin fibroblasts of patients and controls were cultured to a confluence of 50 to 70% in M199 medium (Life Technologies) supplemented with 10% fetal calf serum and 1% penicillin/streptomycin in a humidified atmosphere containing 5% $CO_2$ at 37°C. Cells were switched to a medium consisting of DMEM supplemented with 4mM L-glutamine (Invitrogen), 10% dialyzed FCS (Gibco), 1mM uridine (Calbiochem), and either 5.5 mM D-glucose (Sigma), or 5.5 mM D-Galactose (Merck). After 50 h of incubation, cells were centrifuged for 10 min at 1000rpm and fixed by gentle addition of 2% glutaraldehyde buffered with 0.1 M sodium cacodylate pH7.4 after removing the medium. After at least 4h fixation, the pellets were very carefully taken off and post-fixed in 1% osmium tetroxide in Palade buffer pH 7.4 with 1% potassium hexacyanoferrate(III)-trihydrate, and after dehydration in ethanol and propylene oxide, embedded in Epon for electron microscopy. Semithin 1$\mu$m thick transverse sections were stained with 1% Toluidine Blue. Ultrathin sections were stained with uranyl acetate and lead citrate and examined on a JEOL 1200 EX\II. Most control cells showed fine diffusely dispersed glycogen. Cells with small darkly staining aggregates of glycogen were counted as positive. For each cell line, more than 110 cells were counted and the percentage of positive cells is presented in Table S3.

### Determination of galactose kinetics in whole blood

Enzymatic determination of galactose levels in whole blood was performed using exogenous galactose dehydrogenase (galactose + $NAD^+$ $\rightarrow$ galactono-1,4-lactone + NADH + $H^+$). Galactose levels were determined in a healthy control after oral consumption of 0.3 g galactose (falcento AG, Kreuzlingen, Switzerland) per kilogram body weight dissolved in 250 ml water. Measurements of galactose levels were made at intervals of 10 minutes during the first hour and at intervals of 30 minutes for additional three hours.

3000 μl of galactose buffer reagent (Instruchemie, Delfzijl, Netherlands), 100 μl of galactose NAD reagent (Instruchemie) and 200 μl of a diluted calibrator (Instruchemie) were mixed and the absorbance against distilled water was read at a temperature of 25 °C and a wavelength of 340 nm (optical density 1).

500 μl of each whole blood sample were added to 1000 μl perchloric acid (0.33 mol/l) for deproteinization and then centrifuged. 200 μl of the supernatant were added to 3000 μl galactose buffer reagent, 100 μl galactose NAD reagent and absorbance was read (optical density 1). After supplementation of 20 μl galactose start reagent and incubation at 25 °C for 15 min, the optical density 2 was measured in both, the calibrator and blood samples.

For manual calculation of the galactose concentration the following formula was used:

$$galactose\ (mmol/l) = \frac{\Delta O.D.\ control\ or\ sample}{\Delta O.D.\ calibrator} \times concentration\ of\ calibrator$$

**Clinical phenotype**

**Bifid uvula,** a clinical manifestation atypical for CDG, was present in 16 patients, in two cases with cleft palate but without cleft lips.

**Liver** biopsy was performed in five patients showing steatosis, cholestasis, slight fibrosis and/or bile duct atrophy. An increased amount of glycogen was observed in only two of these patients by PAS staining. In one of these biopsies, electron microscopy revealed glycogen particles in hepatocytes, but much less than in other glycogen storage disorders (Fig. 7).

**Heart:** Three girls with severe DCM (2, 5.2, 6, numbers referring to Table S1) were temporarily listed for cardiac transplantation, but, surprisingly, cardiomyopathy in the first two improved with time. In patient 5.2 and males 3, 7, 14, cardiac arrest occurred, in 5.2 in the context of anesthesia (resuscitated), and in 3 at age 13 induced by exercise (running for his school bus). After resuscitation, he developed epilepsy, a severe ventilation disorder and had episodes of thrombosis. Patients 7 and 14 died at age 8 and 10, respectively, from a cerebrovascular accident and respiratory failure. Both suffered from end-stage DCM.

Electrocardiograms, where available, were checked for arrhythmias associated with DCM, but none were seen. In patient 2, infantile dilated cardiomyopathy (DCM) was diagnosed at 5 months; the lowest measured fractional shortening (FS) of 5% in this patient improved spontaneously to 13% and during complex carbohydrate supplementation to 24%. In patient 5.2, at 16 months, after anesthesia for cleft palate surgery, cardiac arrest occurred. Shortly thereafter, DCM was diagnosed and at age 7 heart transplantation was recommended. However, her heart recovered significantly, with an adult FS at different timepoints of 24% - 33%. Her brother, patient 5.1, had a slightly enlarged left ventricle with normal function (FS 31%).

**Growth:** In several patients with **growth retardation,** where measured, IGF-1/IGFBP3 concentrations around the lower range of normal were observed, with normal or high excretion of growth hormone after provocation with arginine or insulin.

**Other hormones:** Low cortisol and ACTH concentrations were recorded in 2 patients. Activities of coagulation factors ATIII, factor XI, protein C, and protein S were reduced in half of the patients. In addition TSH, which shares one subunit with LH and FSH (described herein, reduced in two girls and associated with hypogonadotropic hypogonadism) was also observed to be low in several cases. All these mentioned hormones and coagulation factors are glycoproteins and have been reported to be affected in patients with CDGs.

**Pseudocholinesterase** (PCHE), a serum glycoprotein produced by liver, for which deficiencies may be associated with prolonged recovery after anesthesia, was measured in several patients and often found to be below the 2.5th percentile. The level was proportional to general glycosylation efficiency as estimated from transferrin glycosylation.

**Sugar metabolite and glycogen quantification**

Determination of **galactose-1-phosphate** (Gal-1-P) levels revealed in the mean 3-fold elevated levels in PGM1-deficient fibroblasts vs. controls (14.4-24.1 nmol/mg protein, mean 18.77, n=6, in PGM1-deficient cells; 3.8-12.1, mean 6.96, n=7, in controls) but normal levels in erythrocytes. The normal Gal-1-P in erythrocytes is likely to be explained by the high PGM2 activity in erythrocytes. In agreement with this interpretation, glycosylation of erythrocyte band 3 glycoprotein was normal (data not shown).

**Glucose-1-P** content was about 10-fold higher in patient fibroblasts than in controls (mean $1.9 \pm 0.5$ nmol/mg protein, n=9, vs. $0.22 \pm 0.12$ nmol/mg protein, n=6), whereas glucose-6-P (n=9 vs. n=6) was normal in patient fibroblasts.

An increased **ratio of UDP-Glc:UDP-Gal** in patient fibroblasts vs. controls ($3,6 \pm 0.58$, n = 5, vs. $2,4 \pm 0.25$, n = 2) suggests that UDP-Gal might be limiting for complex N-glycan synthesis. The data are consistent with a glycogenolytic state of the cells, where Glc-1-P derived from glycogen is increased (due to the PGM1 defect it is not converted to Gluc-6-P). Via the GALT reaction, a portion of the Glc-1-P will react with residual UDP-Gal to increase UDP-Glc and Gal-1-P, leading to the observed metabolite changes in patients vs. control. The differences were mainly due to UDP-Gal with a level of 1150 ± 240 pmol/mg protein, n = 2, controls, vs. 710 ± 250 pmol/mg protein, n = 5, patient cells. UDP-Glc was not significantly different, 2720 ± 280 pmol/mg protein, n = 2, controls, vs. 2580 ± 1050 pmol/mg protein, n = 5, patient cells.

Adding galactose and uridine to PGM1-deficient cells normalized the ratio (n=4) and essentially improved glycosylation (Fig. 14), but left the ratio in control fibroblasts normal (n=3).

**Glycogen** levels in patient fibroblasts (137 ± 62 nmol/mg protein, n=5) were comparable to controls (118 nmol/mg protein, n=2). In addition, analysis of glycogen aggregates was studied by electron microscopy in fibroblasts. Culturing of cells in the presence of either 5.5 mM glucose or 5.5 mM galactose showed no accumulation of glycogen particles in PGM1-deficient fibroblasts (Table S3).

## Lack of effect of PGM1 deficiency on erythrocytes and the brain

Nearly all CDGs show multisystem manifestations, including effects on brain development. In contrast, PGM1-deficient patients have normal neurological function, which is likely a consequence of the presence of alternate enzymes providing PGM function in the brain. Phosphoglucomutases are a family of isoenzymes. PGM1 is expressed ubiquitously and is responsible for 80-95% of the total phosphoglucomutase activity in most tissues and even more in liver, cardiac muscle and white blood cells.[18] PGM2 is responsible for 50% of the total phosphoglucomutase activity in erythrocytes.[18] In brain, PGM2[19,20] and phosphomannomutase 1[21] account for the PGM activity, which is consistent with the absence of a neurological phenotype in PGM1-deficient patients.

## Effect of reduced glycosylation on gonadotropin function: LH and FSH

The gonadotropins LH, FSH and their respective receptors are glycoproteins. LH and FSH are both heterodimers, that have in common a chain "C" with 2 N-glycosylation sites, while the β chains specific for the hormones have 1 (LH-β) and 2 (FSH-β) N-glycosylation sites (e.g. reviewed by Huhtaniemi[22]). There is no specific selection system in the glycosylation machinery that would lead, in patients with PGM1-deficiency, to preferential glycosylation of some specific proteins during the glycosylation process, but excluding LH, FSH etc (see Fig. 1 describing the general process of glycosylation; in addition Fig. 16 on the effect of galactose supplementation, demonstrated that total serum glycoproteins are affected by underglycosylation and that this is reversed by galactose). Thus our data and all evidence from the previous literature suggest that underglycosylation of LH, FSH and their receptors occurs in PGM1-deficiency. The essential question of whether this has functional consequences has also been addressed in literature, and it has been reported that the half-life of underglycosylated LH and FSH is reduced[22]. The essential mechanism impeding LH function, however, is that underglycosylated LH is still able to interact with the LH-receptor, but instead of regular signaling further signaling is blocked.[23]

For FSH, Galway AB et al. demonstrated that FSH molecules that have truncated glycans that lack only the sialic acids, or those from patients with an early block of CDG-I-type (MGAT1- deficiency) leading to only partially synthesized glycans, are still able to bind the receptor and elicit a response in vitro.[24] However, they demonstrated that injection into rats resulted in readily measured estrogen production with wild type FSH, but no response with the variant FSH forms. In a bioactivity assay, injection of wild type FSH resulted in a strong signal for 6-12 hours, while injected variant forms showed no signal at 3, 6, 12, and 24 hours. Thus, the activity half-life of underglycosylated FSH is significantly reduced.

Since we were able to demonstrate an improvement in general glycosylation efficiency with galactose supplementation, our data in combination with evidence from literature show, that the observed onset of puberty soon after galactose supplementation in both hypogonadotropic hypogonadic females is (very likely) a direct consequence of improved glycosylation of LH and FSH, improving both the half-life and the in vivo function of these hormones.

## Effect of reduced glycosylation on IGF1, IGFBP3, IGF-receptor and growth

Fifteen of 19 patients had short stature with height below the 5th percentile and showed reduced insulin-like growth factor 1 (IGF1) and its binding protein (IGFBP3).The physiologically relevant ternary complex is composed of IGF-1, IGFBP3 and the acid labile subunit (ALS). Miller et al. observed, by analyzing serum from 12 CDG-children (vs. 11 controls), significantly reduced serum levels of IGF-1, IGFBP3 and ALS.[25] While IGF-1 is a 70 amino acid peptide that is not glycosylated, most IGF-1 is bound in a functional ternary complex with IGFBP3 and ALS. The latter two carry 3 and 7 N-glycosylation sites respectively. Underglycosylation reduces the stability, the half-life and finally the concentration to about 50% of controls. The CDG-children showed significant growth retardation. While Niehues earlier demonstrated that mannose will correct glycosylation deficiency in CDG-Ib disease[26], Miller showed that mannose supple-

mentation in CDG-Ib not only improves the glycosylation and normalizes the IGF-1, IGFBP3 and ALS levels, but also elicits a catch-up growth into the normal range. The mechanism leading to the growth effect, however, is not only the increased IGF1 level, but also essential improvement of the IGF1-receptor, a glycoprotein with 32 N-glycosylation consensus sites. As demonstrated by Carlberg et al. the IGF-1 receptor must be N-linked glycosylated in order to be translocated and expressed at the cell surface.[27]

Thus, supplementation of mannose in CDG-Ib significantly improves N-glycosylation, and in this way it restores IGF-1/IGF1-receptor function and normal growth.

In PGM1-deficiency, we have shown that IGF1 is reduced, as in CDG-Ib children. There is no reason to assume that ALS, IGFBP3 or IGF1-receptor should be exceptions regarding glycosylation if there is a general defect in glycosylation, as in PGM1-deficiency. Since we have demonstrated that galactose can improve significantly glycosylation, the glycoslyation of the mentioned proteins involved in IGF1-function can be corrected by galactose supplementation and that, as in CDG-Ib, improved glycosylation will also improve growth.

**Dilated cardiomyopathy: loss of functional interaction of PGM1 and ZASP**

The involvement of heart due to dilated cardiomyopathy (DCM) was one of the most clinically important phenotypes associated with *PGM1* defects. Five of 19 patients suffered DCM and three of them experienced a cardiac arrest; one additional patient had a cardiac arrest due to vigorous exercise. The observation that loss of phosphoglucomutase 1 function can be associated with DCM has been made earlier by Arimura et al. Previously, Vatta et al. discovered that mutations in exon 4, 6 or 10 of the Z-disc protein ZASP were associated with dilated cardiomyopathy (DCM).[28] Arimura observed that most ZASP mutations related to DCM occured in exon 4 or 10.[29] They reported that ZASP has alternatively spliced isoforms and that exons 4 and 10 are preferentially expressed in the heart, while transcripts containing exon 6 are more specific for skeletal muscles. Since production of ZASP protein was not affected by some mutations in exons 4 and 6, they started a search for interaction with other proteins that might be disturbed by the mutations. They identified PGM1 binding to regions in ZASP encoded by both exons 4 and 10. They concluded that the lack of local presence of PGM1 in the Z-disc is involved in the pathogenesis of DCM.[29] Our study demonstrates that this is likely case, since defects in the PGM1 itself are also associated with DCM. Although there is a marked reduction of PGM1 activity in all patients, the clinical phenotype is variable. Severe DCM has considerably improved over time in two patients, suggesting that age, lifestyle, and nutrition may have a modulating impact. In cultured cardiomyocytes of rats, PGM1 is normally diffusely distributed in cytoplasm and not bound to ZASP.[29] Only after creation of defined stress conditions, by removing glucose or serum from the culture medium, is PGM1 bound to ZASP in the Z-disk. Anaerobic conditions in which β-oxidation is not possible may require ZASP-PGM1 interaction, and the hypoglycemic periods observed in our patients may contribute as well. However, stabilizing glucose homeostasis by complex carbohydrates and restricting exercise to below the aerobic threshold should be beneficial.

If DCM is progressive, heart transplantation may be life-saving. Since the expression of normal PGM1 in the transplanted heart will restore locally the normal PGM1-ZASP-interaction, it can be assumed that the new heart will be protected from DCM in the PGM1-deficient patient.

**Table S1. Biochemical and Clinical Features in PGM1 Deficiency**

| Patient | 1.1 | 1.2 | 2 | 3 | 4 | 5.1 | 5.2 | 6 | 7 | 8 | 9 | 10 | 11 | 12 | 13.1 | 13.2 | 14 | 15 | 16 |
|---|---|---|---|---|---|---|---|---|---|---|---|---|---|---|---|---|---|---|---|
| **Trait** | | | | | | | | | | | | | | | | | | | |
| Current age/sex | 12/m | 17/m | 19/f | 33/m | 19/m | 26/m | 20/f | 16/f | 8/m | 43/m | 23/f | 9/f | 11/m | 9/m | 5/m | 6/m | 10/m | 3/f | 30/f |
| PGM1 defect † cDNA | 1547T>C | | 988G>C 1129G>A | 787G>T 788A>G | 122A>G 1495C>T | 1145-222G>T | | 1507C>T | 361G>C | 343A>G 1145-1G>C | 122A>G 871G>A | 112A>T | 787G>T 1551C>A | 55A>G 1600-523G>A | 184G>C | | 1162G>A 1547T>C | 551delT | 787G>T 661delC |
| protein | L516P | | G330R E377K | D263Y D263G | Q41R R499X | G382Vfs*23 | | R503X | G121R | T115A Splice | Q41R G291R | N38Y | D263Y Y517X | T19A M535Pfs*60 | D62H | | E388K L516P | F184Sfs*9 | D263Y R221Vfs*13 |
| activity § (% of controls) | 4.4 | NA | 1.3 | 2.4 | 2.5 | 2.2 | 2.8 | 7.7 | 6.6 | 1.2 | 12 | 3.1 | 2.8 | 3.3 | 2.1 | 2.8 | 1.2 | NA | 0.3 |
| **Head** bifid uvula (BU) cleft palate (CP) Pierre-Robin sequence (PRS) | CP,BU | CP, BU | CP,BU & PRS | BU | - | CP, BU | CP, BU & PRS | CP, BU & PRS | CP, BU & PRS | | | CP, BU & PRS | CP, BU & PRS | CP, BU & PRS | CP, BU & PRS | CP, BU & PRS | CP, BU | CP, BU | CP, BU |
| **Heart** dilated cardio-myopathy | - | - | severe FS 5% | - | - | LV slightly enlarged | severe FS 7% | severe FS 5% | severe FS 5% | - | - | - | - | - | - | - | severe FS <10% | - | - |
| cardiac arrest | - | - | - | yes, resuscitated | - | - | yes, resuscitated | - | yes, deceased | - | - | - | - | - | - | - | yes, deceased | - | - |
| **Skeletal muscle** | exercise intol. | exercise intol. | exercise intol. | NA | - | exercise intol. | exercise intol. | - | muscle weakness | exercise intol. | - | - | muscle weakness | exercise intol. | - | - | - | - | exercise intol. |
| Max. CK (U/l) | 789 | 380 | 50 000 | NA | <300 | 10 000 | 7942 | 10 000 | 250 | 10 000 | <300 | 3000 | <300 | 2000 | 200 | 200 | NA | NA | 10 000 |
| Rhabdomyolysis (R) Malignant hyperthermia (M) | - | - | R, M | - | - | R | - | M, R | - | R | - | - | - | - | - | - | - | - | R |
| **Liver** AST, ALT ‡ increased | Yes | Yes | Yes | Yes | Yes | Yes | Yes | Yes | Yes | Yes | Yes | Yes | Yes | Yes | Yes | Yes | Yes | Yes | Yes |
| biopsy results € | steatosis mild fibrosis | - | increased glycogen, steatosis | - | cholestasis, fibrosis, steatosis | - | - | increased glycogen, steatosis | - | - | - | - | cholestasis, steatosis | - | - | - | - | - | - |
| Lowest blood glucose (mM) | 2.6 | 2.8 | 1.0 | 1.9 | 1.8 | 2.8 | 3.2 | 1.8 | 2.6 | 4.2 | 1.6 | <2.7 | 2.4 | 1.4 | 2.1 | 2.4 | 2.6 | 2.4 | 3.6 |
| **Growth ¥ (height percentile: "pc")** | <3 pc | 3 pc | <5 pc | < 3 pc | 50 pc | 3 pc | <3 pc | <3 pc | <3 pc | 90 pc | 75 pc | 10-15 pc | <5 pc | <5 pc | <5 pc | <5 pc | <5 pc | <3 pc | <3 pc |
| **Endocrine** LH (ref. 0.6 - 16 U/L) before galactose | - | - | 0,6 | - | - | - | - | 0.5 | - | - | - | - | - | - | - | - | - | - | - |
| after galactose # | - | - | 3.1-3.5 | - | - | - | - | 4.0-6.2 | - | - | - | - | - | - | - | - | - | - | - |
| FSH (ref. 3.3 - 19 U/L) before galactose | - | - | 2 | - | - | - | - | 2.9 | - | - | - | - | - | - | - | - | - | - | - |
| after galactose | - | - | 5.4-6.5 | - | - | - | - | 4.9-10 | - | - | - | - | - | - | - | - | - | - | - |

**Table S1. Biochemical and Clinical Features in PGM1 Deficiency.**

Families 1, 5, and 13 each included an affected sib pair (1.1, 1.2 etc.). † PGM1 defects: Mutations were numbered based on NM_002633.2 (cDNA) and NG_016966.1 (genomic DNA). Where biological parents were available, both carried an allele of their affected children (7 and 13.1/13.2 were adopted, biological parents not available). $ PGM1 activity was assayed in fibroblasts or leukocytes or from muscle biopsies (patients 8 and 16) and expressed as percentage of the mean control. Effects of mutations on function, structure and expression of mRNA, and on protein levels are detailed in the Figures 10, 11, 12. ‡ ASAT and ALAT levels were mildly elevated from just above normal to 500 U/l (measured in the absence of rhabdomyolysis). € Liver biopsies were evaluated by light microsopy and electron microscopy (Fig. 7). ¥ Growth: All but one of patients with a split uvula also had short stature (< 5th percentile in 15) but all three with a normal uvula had also normal height. # Hormones after galactose: measurements between month 2 - 11 after starting galactose supplementation. FS, fractional shortening (patients' lowest ever measured values); CK, creatine kinase; AST, aspartate aminotransferase; ALT, alanine aminotransferase; LH, luteinizing hormone; FSH, follicle-stimulating hormone; NA, data not available.

**Table S2. PCR Primers and Conditions.**

For sequencing reactions, each forward primer was tagged at the 5'site with the universal primer TGT AAA ACG ACG GCC AGT ("F-") (SEQ ID NO: 12). Each reverse primer was tagged at the 5'site with CAG GAA ACA GCT ATG ACC ("R-") (SEQ ID NO: 13). PCR-conditions were: initial denaturation 5 min. 94°C, cycling 35 x (1 min. 94°C, 1 min. at annealing temperature given in tables below, 1.5 min. 72°C), 10 min. 72°C.

**Table S2.1 Genomic DNA**

| Exon | Primer sequence (5'to 3') | PCR conditions (annealing temperature, +/-Q: Q-solution from Qiagen, Hilden, Germany) |
|---|---|---|
| Ex1 variant 1 | F-GTC CCT TTA AGG AGG AGG GC (SEQ ID NO: 14) R-GAG TCA GGC GAG CAG GTC TG (SEQ ID NO: 15) | 67°C; +Q |
| Ex1 variant 2 | F-CTT TGT GTG TGG GTG CGA GT (SEQ ID NO: 16) R-CTT AAG ACC GGG AGT GTG CT (SEQ ID NO: 17) | 67°C; +Q |
| Ex2 | F-CCT TAC AGT GCA CAC AG (SEQ ID NO: 18) R-GAA GCA AAG AGC ACC TCC CT (SEQ ID NO: 19) | 65°C; +Q |
| Ex3 | F-CCA TGC GCA AAT CTT CC (SEQ ID NO: 20) R-TCA TGC AGC TGA TCC CTG GC (SEQ ID NO: 21) | 65°C; +Q |
| Ex4 | F-ATC CCC TGT GTG CCT CAT CC (SEQ ID NO: 22) R-CTT ACC ACG AGG AGT GTC TC (SEQ ID NO: 23) | 67°C; -Q |
| Ex5 | F-CTC CAC TAG TCC CTG AAC AC (SEQ ID NO: 24) R-GCA TCT AGA ACT CAC CCA GC (SEQ ID NO: 25) | 67°C; -Q |
| Ex6 | F-GCC TTC GGC CCA ATG GTA CT (SEQ ID NO: 26) R-GAG AGG GGC ATT GAA TCC AG (SEQ ID NO: 27) | 67°C; -Q |

(continued)

| Exon | Primer sequence (5'to 3') | PCR conditions (annealing temperature, +/-Q: Q-solution from Qiagen, Hilden, Germany) |
|---|---|---|
| Ex7 | F-TTC TTC TTC CGC CTT CTC TG (SEQ ID NO: 28) R-TCC CTG CAG ACT CAA TGA CC (SEQ ID NO: 29) | 67°C; -Q |
| Ex8 | F-CAG CAA GGG GGA ACT TTG TC (SEQ ID NO: 30) R-CAT GTC CAC CCA ATG GAC AC (SEQ ID NO: 31) | 67°C; -Q |
| Ex9 | F-GTT CCA TCC CCA GTG ACT GA (SEQ ID NO: 32) R-GTG GGA CAG CAG AAA ACC AG (SEQ ID NO: 33) | 67°C; -Q |
| Ex10 | F-CAC CCA GCA TCA CTG AGG TC (SEQ ID NO: 34) R-CAG GCT TGA CCT CCT GGG CT (SEQ ID NO: 35) | 65°C; +Q |
| Ex11 | F-GCA GAG GAA GAT GTC AGG AG (SEQ ID NO: 36) | 65°C; -Q |
|  | R-CAG CTC CCA AGG GCA TCT TG (SEQ ID NO: 37) |  |

**Table S2.2. cDNA**

| I | F-CCT TTC CCC TCC CGC CGG AC (SEQ ID NO: 38) R-GCA CCG AGT TCT TCA CAG AG (SEQ ID NO: 39) | 65°C; +Q |
|---|---|---|
| II | F-CTG GGC CAA ACC GAC TGA AG (SEQ ID NO: 40) R-TGG ATG GGT CCG GGG GTG (SEQ ID NO: 41) | 65°C; +Q |

**Table S3. Glycogen Content in Patient Fibroblasts**

|  |  | % of cells with glycogen aggregates |
|---|---|---|
| 5.5 mM Glucose | Control 1 | 17% |
|  | Control2 | 16% |
|  | PGM1-6 | <1% |
|  | PGM1-7 | <1% |
| 5.5 mM Galactose | Control 1 | 2% |
|  | Control 2 | 4% |
|  | PGM1-6 | <1% |
|  | PGM1-7 | <1% |

Fibroblasts were prepared for evaluation of glycogen by electron microscopy as described above. The presence of glycogen in control and patient fibroblasts cultured in the presence of either glucose or galactose was assessed by electron microscopy. Cells (of 115-160 total) with small darkly staining aggregates of glycogen were counted as positive. Aggregates are typically more common in glycogen storage diseases (picture for hepatocytes s. Fig. 7, panel C, D).

At a first glance these results might give the impression that glycogen is even reduced in PGM1 deficient patients. However, cells in culture contain glycogen of different quality. The glycogen counted by electron microscopy (this table) has stronger staining aggregate grains only. It should be noted that the values are % of cells containing any such aggregates, i.e. more than 80 % of control cells and almost all PGM1-cells didn't show such aggregates, therefore the majority of the normal cells were comparable with PGM1-cells. Glycogen measurement by a biochemical method measured all glycogen instead, including diffuse glycogen, not gathered in aggregates, revealing that the total glycogen amount is not significantly different - only the number of cells with stronger staining aggregates seems in microscopy somewhat lower in the patients. Since this type of aggregates is significantly increased in patients with glycogen storage diseases, it was checked here.

**Table S4. Range of GALT-, PGM-, and G6PDH-Activities in 8 Patients with PGM1-Deficiency and in Healthy Newborns.**

| Whereas GALT and G6PDH do not show clear discrimination between PGM1-deficient patients and controls, our modified assay shows highly significant differences of PGM activity in patients and controls. Design of the assay is described in Fig. 6. The addition of PGM from rabbit heart restored the GALT-activity in the DBS of PGM-deficient patients to normal values. | | | | |
|---|---|---|---|---|
| | GALT (U/dL) | GALT (U/dL) + PGM* | PGM (U/dL) | G6PDH (U/dL) |
| PGM def. (n=8) | 1.9-6.5 | 6.4-17.4 | 7.8-17.1 | 139.4 - 331.2 |
| Controls | 3.4 - 40.7 (n = 2017) (median = 13.9) | 11.9 - 15.3 (n = 4) | 17.5 - 96.2 (n = 2017) (median = 49.3) | 46.1 - 559.5 (n = 1737) (median = 292.0) |
| * = PGM from rabbit muscle was added to the DBS in the GALT assay. | | | | |

**References to EXAMPLES II**

1. Abecasis GR, Cherny SS, Cookson WO, Cardon LR. Merlin--rapid analysis of dense genetic maps using sparse gene flow trees. Nat Genet 2002;30:97-101.

2. Roach JC, Glusman G, Smit AF, et al. Analysis of genetic inheritance in a family quartet by whole-genome sequencing. Science 2010;328:636-9.

3. Nachman MW, Crowell SL. Estimate of the mutation rate per nucleotide in humans. Genetics 2000;156:297-304.

4. Timal S, Hoischen A, Lehle L, et al. Gene identification in the Congenital Disorders of Glycosylation type I by whole-exome sequencing. Hum Mol Genet 2012;21:4151-61.

5. Hoischen A, van Bon BW, Rodriguez-Santiago B, et al. De novo nonsense mutations in ASXL1 cause Bohring-Opitz syndrome. Nat Genet 2011;43:729-31.

6. Rozen S, Skaletzky HJ. Primer3 on the WWW for general users and for biologist programmers. In: Krawetz S, S. M, eds. Bioinformatics Methods and Protocols: Methods in Molecular Biology. Totowa, NJ: Humana Press; 2000:365-86.

7. Lowry OH, Rosebrough NJ, Farr AL, Randall RJ. Protein measurement with the Folin phenol reagent. J Biol Chem 1951;193:265-75.

8. Babovic-Vuksanovic D, O'Brien JF. Laboratory diagnosis of congenital disorders of glycosylation type I by analysis of transferrin glycoforms. Mol Diagn Ther 2007;11:303-11.

9. Wada Y, Tajiri M, Yoshida S. Hydrophilic affinity isolation and MALDI multiple-stage tandem mass spectrometry of glycopeptides for glycoproteomics. Anal Chem 2004;76:6560-5.

10. He P, Ng BG, Losfeld ME, Zhu W, Freeze HH. Identification of intercellular cell adhesion molecule 1 (ICAM-1) as a hypo-glycosylation marker in congenital disorders of glycosylation cells. J Biol Chem 2012.

11. Losfeld ME, Soncin F, Ng BG, K. S, Freeze HH. A sensitive Green Fluorescent Protein biomarker of N-glycosylation site occupancy. FASEB J 2012;in press.

12. Rabina J, Maki M, Savilahti EM, Jarvinen N, Penttila L, Renkonen R. Analysis of nucleotide sugars from cell lysates by ion-pair solid-phase extraction and reversed-phase high-performance liquid chromatography. Glycoconj J 2001;18:799-805.

13. Nakajima K, Kitazume S, Angata T, et al. Simultaneous determination of nucleotide sugars with ion-pair reversed-phase HPLC. Glycobiology 2010;20:865-71.

14. Shin YS. Galactose metabolites and disorders of galactose metabolism. In: Hommes FA, ed. Techniques in diagnostic human biochemical genetics. New York: Wiley-Liss; 1991:267-83.

15. Donthi RV, Epstein PN. Altering and analyzing glucose metabolism in perfused hearts of transgenic mice. Methods Mol Med 2007;139:151-61.

16. Passonneau JV, Lauderdale VR. A comparison of three methods of glycogen measurement in tissues. Anal Biochem 1974;60:405-12.

17. Sickmann HM, Schousboe A, Fosgerau K, Waagepetersen HS. Compartmentation of lactate originating from glycogen and glucose in cultured astrocytes. Neurochem Res 2005;30:1295-304.

18. McAlpine PJ, Hopkinson DA, Harris H. The relative activities attributable to the three phosphoglucomutase loci (PGM1, PGM2, PGM3) in human tissues. Ann Hum Genet 1970;34:169-75.

19. Quick CB, Fisher RA, Harris H. A kinetic study of the isozymes determined by the three human phosphoglucomutase loci PGM1, PGM2, and PGM3. Eur J Biochem 1974;42:511-7.

20. Maliekal P, Sokolova T, Vertommen D, Veiga-da-Cunha M, Van Schaftingen E. Molecular identification of mammalian phosphopentomutase and glucose-1,6-bisphosphate synthase, two members of the alpha-D-phosphohexomutase family. J Biol Chem 2007;282:31844-51.

21. Pirard M, Achouri Y, Collet JF, Schollen E, Matthijs G, Van Schaftingen E. Kinetic properties and tissular distribution of mammalian phosphomannomutase isozymes. Biochem J 1999;339 (Pt 1):201-7.

22. Huhtaniemi IT. Polymorphism of gonadotropin action: clinical implications. Asian J Androl 2000;2:241-6.

23. Sairam MR, Fleshner P. Inhibition of hormone-induced cyclic AMP production and steroidogenesis in interstitial cells by deglycosylated lutropin. Mol Cell Endocrinol 1981;22:41-54.

24. Galway AB, Hsueh AJ, Keene JL, Yamoto M, Fauser BC, Boime I. In vitro and in vivo bioactivity of recombinant human follicle-stimulating hormone and partially deglycosylated variants secreted by transfected eukaryotic cell lines. Endocrinology 1990;127:93-100.

25. Miller BS, Khosravi MJ, Patterson MC, Conover CA. IGF system in children with congenital disorders of glycosylation. Clin Endocrinol (Oxf) 2009;70:892-7.

26. Niehues R, Hasilik M, Alton G, et al. Carbohydrate-deficient glycoprotein syndrome type lb. Phosphomannose isomerase deficiency and mannose therapy. J Clin Invest 1998;101:1414-20.

27. Carlberg M, Larsson O. Stimulatory effect of PDGF on HMG-CoA reductase activity and N-linked glycosylation contributes to increased expression of IGF-1 receptors in human fibroblasts. Exp Cell Res 1996;223:142-8.

28. Vatta M, Mohapatra B, Jimenez S, et al. Mutations in Cypher/ZASP in patients with dilated cardiomyopathy and left ventricular non-compaction. J Am Coll Cardiol 2003;42:2014-27.

29. Arimura T, Inagaki N, Hayashi T, et al. Impaired binding of ZASP/Cypher with phosphoglucomutase 1 is associated with dilated cardiomyopathy. Cardiovasc Res 2009;83:80-8.

30. Dai JB, Liu Y, Ray WJ, Jr., Konno M. The crystal structure of muscle phosphoglucomutase refined at 2.7-angstrom resolution. J Biol Chem 1992;267:6322-37.

31. Shackelford GS, Regni CA, Beamer LJ. Evolutionary trace analysis of the alpha-D-phosphohexomutase superfamily. Protein Sci 2004;13:2130-8.

32. Gururaj A, Barnes CJ, Vadlamudi RK, Kumar R. Regulation of phosphoglucomutase 1 phosphorylation and activity by a signaling kinase. Oncogene 2004;23:8118-27.

33. Milstein C, Sanger F. An amino acid sequence in the active centre of phosphoglucomutase. Biochem J 1961;79:456-69.

34. Hindle AG, Karimpour-Fard A, Epperson LE, Hunter LE, Martin SL. Skeletal muscle proteomics: carbohydrate metabolism oscillates with seasonal and torpor-arousal physiology of hibernation. Am J Physiol Regul Integr Comp Physiol 2011;301:R1440-52.

35. March RE, Putt W, Hollyoake M, et al. The classical human phosphoglucomutase (PGM1) isozyme polymorphism is generated by intragenic recombination. Proc Natl Acad Sci U S A 1993;90:10730-3.

36. Maquat LE. Nonsense-mediated mRNA decay in mammals. J Cell Sci 2005;118:1773-6.

37. Schadt EE, Molony C, Chudin E, et al. Mapping the genetic architecture of gene expression in human liver. PLoS Biol 2008;6:e107.

38. Kutalik Z, Benyamin B, Bergmann S, et al. Genome-wide association study identifies two loci strongly affecting transferrin glycosylation. Hum Mol Genet 2011;20:3710-7.

39. Perez B, Medrano C, Ecay MJ, et al. A novel congenital disorder of glycosylation type without central nervous system involvement caused by mutations in the phosphoglucomutase 1 gene. J Inherit Metab Dis 2012.

40. Bloom JW, Madanat MS, Ray MK. Cell line and site specific comparative analysis of the N-linked oligosaccharides on human ICAM-1des454-532 by electrospray ionization mass spectrometry. Biochemistry 1996;35:1856-64.

41. Vanderschaeghe D, Laroy W, Sablon E, et al. GlycoFibroTest is a highly performant liver fibrosis biomarker derived from DNA sequencer-based serum protein glycomics. Mol Cell Proteomics 2009;8:986-94.

**Claims**

1. A method for diagnosing a mixed congenital disorder of glycosylation (CDG) caused by a deficiency of phosphoglucomutase-1 (PGM1) comprising

   (a) adding separately (i) glucose-1-phosphate (G1P) and (ii) glucose-6-phosphate (G6P) in a Beutler test format to a sample obtained from a subject; and
   (b) comparing the signal from (i) and (ii),
   wherein the subject suffers from said CDG, if the signal (ii) exceeds the signal of (i).

2. The method of claim 1, wherein said mixed congenital disorder of glycosylation (CDG) caused by a deficiency of phosphoglucomutase-1 (PGM1) is PGM1-CDG.

3. The method of claim 1 or 2, wherein the subject is a newborn.

4. The method of any one of claims 1-3, wherein the signal is fluorescence.

5. The method of any one of the preceding claims, wherein the sample is a blood sample.

6. The method of any one of the preceding claims, wherein the sample is a red blood sample.

7. The method of claim 6, wherein the red blood sample is whole blood, washed red blood cells, a hemolysate made from red blood cells, and/or dried whole blood on a physical support.

8. The method of any one of the preceding claims, further comprising adding PGM1, Glucose-6-phosphate-Dehydrogenase (G6PD), and/or 6-Phosphogluconat-dehydrogenase (6PGDH).

9. The method of any one of the preceding claims, wherein glucose-1,6-bisphosphate is present in the Beutler test format.

10. The method of claim 9, wherein glucose-1,6-bisphosphate is present in the range between about 5 $\mu$M to about 200 $\mu$M.

11. Use of Glucose-1-phospate and Glucose-6-phospate for diagnosing a mixed congenital disorder of glycosylation (CDG) caused by a deficiency of phosphoglucomutase-1 (PGM1).

12. The use of claim 11, further comprising using glucose-1,6-bisphosphate.

13. Use of PGM1 for diagnosing a mixed CDG caused by a deficiency of PGM1, said use comprising subjecting a sample obtained from a subject to a Beutler test in the (i) absence and (ii) presence of exogenously added PGM1, wherein the subject suffers from said CDG, if the signal (ii) exceeds the signal of (i).

14. A kit comprising a Beutler assay format, said Beutler assay format comprising

   i) a support or carrier, preferably Guthrie heel prick test cards, and
   ii) glucose-1-phosphate and glucose-6-phosphate and optionally further glucose-1,6-bisphosphate,

wherein said glucose-1-phosphate and/or glucose-6-phosphate and/or glucose-1,6-bisphosphate is preferably coated onto a solid support.

15. A Beutler assay format comprising

    i) a support or carrier, preferably Guthrie heel prick test cards, and
    ii) glucose-1-phosphate and glucose-6-phosphate and optionally further glucose-1,6-bisphosphate,

wherein said glucose-1-phosphate and/or glucose-6-phosphate and/or glucose-1,6-bisphosphate is preferably coated onto a solid support.


**Patentansprüche**

1. Verfahren zum Diagnostizieren einer durch einen Mangel an Phosphoglukomutase-1 (PGM1) verursachten, gemischten kongenitalen Glykosylierungsstörung (CDG), umfassend

    (a) separates Hinzufügen von (i) Glukose-1-phosphat (G1P) und (ii) Glukose-6-phosphat (G6P) in einem Beutler-Testformat zu einer Probe, die von einem Subjekt erhalten wurde; und
    (b) Vergleichen des Signals von (i) und (ii),
    wobei das Subjekt unter CDG leidet, wenn das Signal (ii) das Signal von (i) überschreitet.

2. Verfahren nach Anspruch 1, wobei die durch einen Mangel an Phosphoglukomutase-1 (PGM1) verursachte, gemischte kongenitale Glykosylierungsstörung (CDG) PGM1-CDG ist.

3. Verfahren nach Anspruch 1 oder 2, wobei das Subjekt ein Neugeborenes ist.

4. Verfahren nach einem der Ansprüche 1-3, wobei das Signal Fluoreszenz ist.

5. Verfahren nach einem der vorhergehenden Ansprüche, wobei die Probe eine Blutprobe ist.

6. Verfahren nach einem der vorhergehenden Ansprüche, wobei die Probe eine rote Blutprobe ist.

7. Verfahren nach Anspruch 6, wobei die rote Blutprobe Vollblut, gewaschene rote Blutkörperchen, ein Hämolysat aus roten Blutkörperchen und/oder getrocknetes Vollblut auf einem körperlichen Träger ist.

8. Verfahren nach einem der vorhergehenden Ansprüche, weiterhin umfassend das Hinzufügen von PGM1, Glukose-6-phosphat-Dehydrogenase (G6PD) und/oder 6-Phosphoglukonat-Dehydrogenase (6 PGDH).

9. Verfahren nach einem der vorhergehenden Ansprüche, wobei Glukose-1,6-bisphosphat in dem Beutler-Testformat vorhanden ist.

10. Verfahren nach Anspruch 9,
wobei Glukose-1,6-bisphosphat in dem Bereich zwischen etwa 5 $\mu$M bis etwa 200 $\mu$M vorhanden ist.

11. Verwendung von Glukose-1-phosphat und Glukose-6-phosphat zum Diagnostizieren einer durch einen Mangel an Phosphoglukomutase-1 (PGM1) verursachten, gemischten kongenitalen Glykosylierungsstörung (CDG).

12. Verwendung nach Anspruch 11, weiterhin umfassend die Verwendung von Glukose-1,6-bisphosphat.

13. Verwendung von PGM1 zum Diagnostizieren einer durch einen PGM1-Mangel verursachten, gemischten CDG, wobei die Verwendung umfasst, dass eine von einem Subjekt erhaltene Probe einem Beutler-Test unterzogen wird, bei (i) Abwesenheit und (ii) Vorhandensein von exogen hinzugefügtem PGM1, wobei der Patient unter CDG leidet, wenn das Signal (ii) das Signal von (i) überschreitet.

14. Kit, umfassend ein Beutler-Assayformat, wobei das Beutler-Assayformat umfasst:

    i) eine Unterlage oder einen Träger, vorzugsweise Guthrie-Testkarten, und

ii) Glukose-1-phosphat und Glukose-6-phosphat und wahlweise weiterhin Glukose-1,6-bisphosphat,

wobei Glukose-1-phosphat und/oder Glukose-6-phosphat und/oder Glukose-1,6-bisphosphat vorzugsweise auf einen festen Träger aufgetragen sind.

**15.** Beutler-Assayformat, umfassend

i) eine Unterlage oder einen Träger, vorzugsweise Guthrie-Testkarten, und
ii) Glukose-1-phosphat und Glukose-6-phosphat und wahlweise weiterhin Glukose-1,6-bisphosphat,

wobei Glukose-1-phosphat und/oder Glukose-6-phosphat und/oder Glukose-1,6-bisphosphat vorzugsweise auf einen festen Träger aufgetragen sind.

**Revendications**

**1.** Procédé pour diagnostiquer une anomalie congénitale mixte de glycosylation (CDG) causée par une déficience de phosphoglucomutase-1 (PGM1) comportant

(a) ajouter séparément (i) du glucose-1-phosphate (G1P) et (ii) du glucose-6-phosphate (G6P) dans un format de test Beutler à un échantillon obtenu d'un sujet; et
(b) comparer le signal de (i) et (ii),
le sujet souffrant de ladite CDG, si le signal (ii) excède le signal de (i).

**2.** Procédé selon la revendication 1, ladite anomalie congénitale mixte de glycosylation (CDG) causée par une déficience de phosphoglucomutase-1 (PGM1) étant PGM1-CDG.

**3.** Procédé selon la revendication 1 ou 2, le sujet étant un nouveau-né.

**4.** Procédé selon l'une quelconque des revendications 1-3, le signal étant de la fluorescence.

**5.** Procédé selon l'une quelconque des revendications précédentes, l'échantillon étant un échantillon de sang.

**6.** Procédé selon l'une quelconque des revendications précédentes, l'échantillon étant un échantillon de sang rouge.

**7.** Procédé selon la revendication 6, l'échantillon de sang rouge étant du sang entier, des globules rouges lavés, un hémolysate produit à partir des globules rouges et/ou du sang entier séché sur un support physique.

**8.** Procédé selon l'une quelconque des revendications précédentes, comportant en outre l'ajout du PGM1, du glucose-6-phosphate-déshydrogénase (G6PD) et/ou du 6-phosphogluconate-déshydrogénase (6PGDH).

**9.** Procédé selon l'une quelconque des revendications précédentes, le glucose-1,6-bisphosphate étant présent dans le format de test Beutler.

**10.** Procédé selon la revendication 9,
le glucose-1,6-bisphosphate étant présent dans la gamme entre environ 5 $\mu$M à environ 200 $\mu$M.

**11.** Utilisation du glucose-1-phosphate et du glucose-6-phosphate pour diagnostiquer une anomalie congénitale mixte de glycosylation (CDG) causée par une déficience de phosphoglucomutase-1 (PGM1).

**12.** Utilisation selon la revendication 11, comportant en outre l'utilisation du glucose-1,6-bisphosphate.

**13.** Utilisation de PGM1 pour diagnostiquer une CDG mixte causée par une déficience de PGM1, ladite utilisation comportant la soumission d'un échantillon obtenu d'un sujet à un test Beutler dans (i) l'absence et (ii) la présence de PGM1 ajoutée de manière exogène, le sujet souffrant de ladite CDG, si le signal (ii) excède le signal de (i).

**14.** Kit comportant un format d'essai Beutler, ledit format d'essai Beutler comportant

i) un appui ou un support, de préférence des cartes du test de Guthrie, et

ii) du glucose-1-phosphate et du glucose-6-phosphate et facultativement en outre du glucose-1,6-bisphosphate,

ledit glucose-1-phosphate et/ou du glucose-6-phosphate et/ou du glucose-1,6-bisphosphate étant de préférence revêtu sur un support solide.

15. Format d'essai Beutler comportant

i) un appui ou un support, de préférence des cartes du test de Guthrie, et

ii) du glucose-1-phosphate et du glucose-6-phosphate et facultativement en outre du glucose-1,6-bisphosphate,

ledit glucose-1-phosphate et/ou du glucose-6-phosphate et/ou du glucose-1,6-bisphosphate étant de préférence revêtu sur un support solide.

**Figure 1**

Figure 2

**Figure 3**

**A  Bifid Uvula**

**B  Symptoms in Patients**

Figure 4

**Figure 5**

**Figure 6**

**Figure 7**

**Figure 8**

Figure 9

Chromosome 2, Lodscore/ Basepairs

Chromosome 5, Lodscore/ Basepairs

Chromosome 6, Lodscore/ Basepairs

Chromosome 7, Lodscore/ Basepairs

**Figure 9 continued**

Chromosome 8, Lodscore/ Basepairs

Chromosome 10, Lodscore/ Basepairs

Chromosome 16, Lodscore/ Basepairs

**Figure 9 continued**

Figure 10

**Figure 11**

**Figure 12**

**Figure 13**

**Figure 14**

Figure 15

**Figure 16**

**Figure 17**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0763132 A

**Non-patent literature cited in the description**

- **QUICK CB ; FISHER RA ; HARRIS H.** A kinetic study of the isozymes determined by the three human phosphoglucomutase loci PGM1, PGM2, and PGM3. *Eur J Bio- chem,* 1974, vol. 42, 511-7
- **STOJKOVIC T ; VISSING J ; PETIT F et al.** Muscle glycogenosis due to phosphoglucomutase 1 deficiency. *N Engl J Med,* 2009, vol. 361, 425-7
- **NIEHUES R ; HASILIK M ; ALTON G et al.** Carbohydrate-deficient glycoprotein syndrome type lb: phosphomannose isomerase deficiency and mannose therapy. *J Clin Invest,* 1998, vol. 101, 1414-20
- **DE JONG G ; VAN NOORT WL ; VAN EIJK HG.** Optimized separation and quantitation of serum and cerebrospinal fluid transferrin subfractions defined by differences in iron saturation or glycan composition. *Adv Exp Med Biol,* 1994, vol. 356, 51-9
- **MANDATO C ; BRIVE L ; MIURA Y et al.** Cryptogenic liver disease in four children: a novel congenital disorder of glycosylation. *Pediatr Res,* 2006, vol. 59, 293-8
- **MOHAMED M ; GUILLARD M ; WORTMANN SB et al.** Clinical and diagnostic approach in unsolved CDG patients with a type 2 transferrin pattern. *Biochim Biophys Acta,* 2011, vol. 1812, 691-8
- **GEHRMANN J ; SOHLBACH K ; LINNEBANK M et al.** Cardiomyopathy in congenital disorders of glycosylation. *Cardiol Young,* 2003, vol. 13, 345-51
- **TIMAL S ; HOISCHEN A ; LEHLE L et al.** Gene identification in the congenital disorders of glycosylation type I by whole-exome sequencing. *Hum Mol Genet,* 2012, vol. 21, 4151-61
- **BIFFI S ; TAMARO G ; BORTOT B ; ZAMBER- IAN S ; SEVERINI GM ; CARROZZI M.** Carbohydrate-deficient transferrin (CDT) as a biochemical tool for the screening of congenital disorders of glycosylation (CDGs). *Clin Biochem,* 2007, vol. 40, 1431-4
- **HE P ; NG BG ; LOSFELD ME ; ZHU W ; FREEZE HH.** Identification of intercellular cell adhesion molecule 1 (ICAM-1) as a hypoglycosylation marker in congenital disorders of glycosylation cells. *J Biol Chem,* 2012, vol. 287, 18210-7

- **LOSFELD ME ; SONCIN F ; NG BG ; SINGEC I ; FREEZE HH.** A sensitive green fluorescent protein biomarker of N-glycosylation site occupancy. *FASEB J,* 2012, vol. 26, 4210-7
- **RÄBINÄ J ; MÄKI M ; SAVILAHTI EM ; JÄRVINEN N ; PENTTILÄ L ; RENKONEN R.** Analysis of nucleotide sugars from cell lysates by ion-pair solid-phase extraction and reversed-phase high-performance liquid chromatography. *Glycoconj J,* 2001, vol. 18, 799-805
- **NAKAJIMA K ; KITAZUME S ; ANGATA T et al.** Simultaneous determination of nucleotide sugars with ion-pair reversed-phase HPLC. *Glycobiology,* 2010, vol. 20, 865-71
- Galactose metabolites and disorders of galactose metabolism. **SHIN YS.** Techniques in diagnostic human biochemical genetics. Wiley-Liss, 1991, 267-83
- **PASSONNEAU JV ; LAUDERDALE VR.** A comparison of three methods of glycogen measurement in tissues. *Anal Biochem,* 1974, vol. 60, 405-12
- **SICKMANN HM ; SCHOUSBOE A ; FOSGERAU K ; WAAGEPETERSEN HS.** Compartmentation of lactate originating from glycogen and glucose in cultured astrocytes. *Neurochem Res,* 2005, vol. 30, 1295-304
- **PEREZ B ; MEDRANO C ; ECAY MJ et al.** A novel congenital disorder of glycosylation type without central nervous system involvement caused by mutations in the phosphoglucomutase 1 gene. *J Inherit Metab Dis,* 2013, vol. 36, 535-42
- **THOMSON WH ; MACLAURIN JC ; PRINEAS JW.** Skeletal muscle glycogenosis: an investigation of two dissimilar cases. *J Neurol Neurosurg Psychiatry,* 1963, vol. 26, 60-8
- **SUGIE H ; KOBAYASHI J ; SUGIE Y et al.** Infantile muscle glycogen storage disease: phosphoglucomutase deficiency with decreased muscle and serum carnitine levels. *Neurology,* 1988, vol. 38, 602-5

- Glycogen storage diseases, types III, IV, and VI. **ILLINGWORTH B ; BROWN DH.** Control of glycogen metabolism (Ciba Foundation Symposium). J. & A. Churchill, 1964, 336-53

- Phosphoglucomutase deficiency as a possible cause of glycogenosis. **ILLINGWORTH BROWN B ; BROWN DH.** Carbohydrate metabolism and its disorders. Academic Press, 1968, 144-6

- **NAKASHIMA H ; SUO H ; OCHIAI J ; SUGIE H ; KAWAMURA Y.** A case of adult onset phosphoglucomutase deficiency. *Rinsho Shinkeigaku,* 1992, vol. 32, 42-7

- **WEINSTEIN DA ; WOLFSDORF JI.** Effect of continuous glucose therapy with uncooked cornstarch on the long-term clinical course of type 1a glycogen storage disease. *Eur J Pediatr,* 2002, vol. 161 (1), S35-S39

- **HUHTANIEMI IT.** Polymorphism of gonadotropin action: clinical implications. *Asian J Androl,* 2000, vol. 2, 241-6

- **MILLER BS ; KHOSRAVI MJ ; PATTERSON MC ; CONOVER CA.** IGF system in children with congenital disorders of glycosylation. *Clin Endocrinol (Oxf),* 2009, vol. 70, 892-7

- **ARIMURA T ; INAGAKI N ; HAYASHI T et al.** Impaired binding of ZASP/Cypher with phosphoglucomutase 1 is associated with dilated cardiomyopathy. *Cardiovasc Res,* 2009, vol. 83, 80-8

- **VATTA M ; MOHAPATRA B ; JIMENEZ S et al.** Mutations in Cypher/ZASP in patients with dilated cardiomyopathy and left ventricular non-compaction. *J Am Coll Car- diol,* 2003, vol. 42, 2014-27

- **ABECASIS GR ; CHERNY SS ; COOKSON WO ; CARDON LR.** Merlin--rapid analysis of dense genetic maps using sparse gene flow trees. *Nat Genet,* 2002, vol. 30, 97-101

- **ROACH JC ; GLUSMAN G ; SMIT AF et al.** Analysis of genetic inheritance in a family quartet by whole-genome sequencing. *Science,* 2010, vol. 328, 636-9

- **NACHMAN MW ; CROWELL SL.** Estimate of the mutation rate per nucleotide in humans. *Genetics,* 2000, vol. 156, 297-304

- **TIMAL S ; HOISCHEN A ; LEHLE L et al.** Gene identification in the Congenital Disorders of Glycosylation type I by whole-exome sequencing. *Hum Mol Genet,* 2012, vol. 21, 4151-61

- **HOISCHEN A ; VAN BON BW ; RODRIGUEZ-SANTIAGO B et al.** De novo nonsense mutations in ASXL1 cause Bohring-Opitz syndrome. *Nat Genet,* 2011, vol. 43, 729-31

- Primer3 on the WWW for general users and for biologist programmers. **ROZEN S ; SKALETZKY HJ.** Bioinformatics Methods and Protocols: Methods in Molecular Biology. Humana Press, 2000, 365-86

- **LOWRY OH ; ROSEBROUGH NJ ; FARR AL ; RANDALL RJ.** Protein measurement with the Folin phenol reagent. *J Biol Chem,* 1951, vol. 193, 265-75

- **BABOVIC-VUKSANOVIC D ; O'BRIEN JF.** Laboratory diagnosis of congenital disorders of glycosylation type I by analysis of transferrin glycoforms. *Mol Diagn Ther,* 2007, vol. 11, 303-11

- **WADA Y ; TAJIRI M ; YOSHIDA S.** Hydrophilic affinity isolation and MALDI multiple-stage tandem mass spectrometry of glycopeptides for glycoproteomics. *Anal Chem,* 2004, vol. 76, 6560-5

- **HE P ; NG BG ; LOSFELD ME ; ZHU W ; FREEZE HH.** Identification of intercellular cell adhesion molecule 1 (ICAM-1) as a hypo-glycosylation marker in congenital disorders of glycosylation cells. *J Biol Chem,* 2012

- **LOSFELD ME ; SONCIN F ; NG BG, K. S ; FREEZE HH.** A sensitive Green Fluorescent Protein biomarker of N-glycosylation site occupancy. *FASEB J,* 2012

- **RABINA J ; MAKI M ; SAVILAHTI EM ; JARVINEN N ; PENTTILA L ; RENKONEN R.** Analysis of nucleotide sugars from cell lysates by ion-pair solid-phase extraction and reversed-phase high-performance liquid chromatography. *Glycoconj J,* 2001, vol. 18, 799-805

- **DONTHI RV ; EPSTEIN PN.** Altering and analyzing glucose metabolism in perfused hearts of transgenic mice. *Methods Mol Med,* 2007, vol. 139, 151-61

- **MCALPINE PJ ; HOPKINSON DA ; HARRIS H.** The relative activities attributable to the three phosphoglucomutase loci (PGM1, PGM2, PGM3) in human tissues. *Ann Hum Genet,* 1970, vol. 34, 169-75

- **QUICK CB ; FISHER RA ; HARRIS H.** A kinetic study of the isozymes determined by the three human phosphoglucomutase loci PGM1, PGM2, and PGM3. *Eur J Biochem,* 1974, vol. 42, 511-7

- **MALIEKAL P ; SOKOLOVA T ; VERTOMMEN D ; VEIGA-DA-CUNHA M ; VAN SCHAFTINGEN E.** Molecular identification of mammalian phosphopentomutase and glucose-1,6-bisphosphate synthase, two members of the alpha-D-phosphohexomutase family. *J Biol Chem,* 2007, vol. 282, 31844-51

- **PIRARD M ; ACHOURI Y ; COLLET JF ; SCHOLLEN E ; MATTHIJS G ; VAN SCHAFTINGEN E.** Kinetic properties and tissular distribution of mammalian phosphomannomutase isozymes. *Biochem J,* 1999, vol. 339, 201-7

- **SAIRAM MR ; FLESHNER P.** Inhibition of hormone-induced cyclic AMP production and steroidogenesis in interstitial cells by deglycosylated lutropin. *Mol Cell Endocrinol,* 1981, vol. 22, 41-54

- **GALWAY AB ; HSUEH AJ ; KEENE JL ; YAMOTO M ; FAUSER BC ; BOIME I.** In vitro and in vivo bioactivity of recombinant human follicle-stimulating hormone and partially deglycosylated variants secreted by transfected eukaryotic cell lines. *Endocrinology,* 1990, vol. 127, 93-100
- **NIEHUES R ; HASILIK M ; ALTON G et al.** Carbohydrate-deficient glycoprotein syndrome type Ib. Phosphomannose isomerase deficiency and mannose therapy. *J Clin Invest,* 1998, vol. 101, 1414-20
- **CARLBERG M ; LARSSON O.** Stimulatory effect of PDGF on HMG-CoA reductase activity and N-linked glycosylation contributes to increased expression of IGF-1 receptors in human fibroblasts. *Exp Cell Res,* 1996, vol. 223, 142-8
- **VATTA M ; MOHAPATRA B ; JIMENEZ S et al.** Mutations in Cypher/ZASP in patients with dilated cardiomyopathy and left ventricular non-compaction. *J Am Coll Cardiol,* 2003, vol. 42, 2014-27
- **DAI JB ; LIU Y ; RAY WJ, JR. ; KONNO M.** The crystal structure of muscle phosphoglucomutase refined at 2.7-angstrom resolution. *J Biol Chem,* 1992, vol. 267, 6322-37
- **SHACKELFORD GS ; REGNI CA ; BEAMER LJ.** Evolutionary trace analysis of the alpha-D-phosphohexomutase superfamily. *Protein Sci,* 2004, vol. 13, 2130-8
- **GURURAJ A ; BARNES CJ ; VADLAMUDI RK ; KUMAR R.** Regulation of phosphoglucomutase 1 phosphorylation and activity by a signaling kinase. *Oncogene,* 2004, vol. 23, 8118-27
- **MILSTEIN C ; SANGER F.** An amino acid sequence in the active centre of phosphoglucomutase. *Biochem J,* 1961, vol. 79, 456-69
- **HINDLE AG ; KARIMPOUR-FARD A ; EPPERSON LE ; HUNTER LE ; MARTIN SL.** Skeletal muscle proteomics: carbohydrate metabolism oscillates with seasonal and torpor-arousal physiology of hibernation. *Am J Physiol Regul Integr Comp Physiol,* 2011, vol. 301, R1440-52
- **MARCH RE ; PUTT W ; HOLLYOAKE M et al.** The classical human phosphoglucomutase (PGM1) isozyme polymorphism is generated by intragenic recombination. *Proc Natl Acad Sci U S A,* 1993, vol. 90, 10730-3
- **MAQUAT LE.** Nonsense-mediated mRNA decay in mammals. *J Cell Sci,* 2005, vol. 118, 1773-6
- **SCHADT EE ; MOLONY C ; CHUDIN E et al.** Mapping the genetic architecture of gene expression in human liver. *PLoS Biol,* 2008, vol. 6, e107
- **KUTALIK Z ; BENYAMIN B ; BERGMANN S et al.** Genome-wide association study identifies two loci strongly affecting transferrin glycosylation. *Hum Mol Genet,* 2011, vol. 20, 3710-7
- **PEREZ B ; MEDRANO C ; ECAY MJ et al.** A novel congenital disorder of glycosylation type without central nervous system involvement caused by mutations in the phosphoglucomutase 1 gene. *J Inherit Metab Dis,* 2012
- **BLOOM JW ; MADANAT MS ; RAY MK.** Cell line and site specific comparative analysis of the N-linked oligosaccharides on human ICAM-1des454-532 by electrospray ionization mass spectrometry. *Biochemistry,* 1996, vol. 35, 1856-64
- **VANDERSCHAEGHE D ; LAROY W ; SABLON E et al.** GlycoFibroTest is a highly performant liver fibrosis biomarker derived from DNA sequencer-based serum protein glycomics. *Mol Cell Proteomics,* 2009, vol. 8, 986-94